# EUROPEAN PATENT SPECIFICATION

(11) **EP 1 752 162 B1**
(45) Date of publication and mention of the grant of the patent: **15.07.2009**
(21) Application number: 05744881.3
(22) Date of filing: 25.05.2005
(51) Int. Cl.: A61K 38/39, A61K 31/79, A61P 29/00, A61L 27/24, A61L 27/16

(54) **COLLAGEN-POLYVINYLPYRROLIDONE FOR THE TREATMENT OF OSTEOARTHRITIS**
KOLLAGEN-POLYVINYLPYRROLIDON ZUR BEHANDLUNG VON OSTEOARTHRITIS
COLLAGENE-POLYVINYLPIRROLIDONE POUR LE TRAITEMENT DE L'OSTEOARTHRITE

(30) Priority: 27.05.2004 MX PA04005080
(43) Date of publication of application: 14.02.2007
(73) Proprietor: Aspid, S.A. de C.V., México, D.F. 03300 (MX)
(72) Inventor: KROTZSCH GOMEZ, Fernando Edgar, México D.F. 03300 (MX); FURUZAWA CARBALLEDA, Guadalupe Janette, México, D.F. 14640 (MX)
(74) Representative: Prinz & Partner
(86) International application number: PCT/MX2005/000041
(87) International publication number: WO 2005/115443

(56) References cited:
- FURUZAWA-CARBALLEDA J. ET AL: 'Mediators of inflammation are down-regulated while apoptosis is up-regulated in rheumatoid arthritis synovial tissue by polymerized collagen' CLIN. EXP. IMMUNOL. vol. 130, 2002, pages 140 - 149, XP008073999
- FURUZAWA-CARBALLEDA J. ET AL: 'Collagen-PVP decreases collagen turnover in synovial tissue cultures from rheumatoid arthritis patients' ANNALA OF THE NEW YORK ACADEMY OF SCIENCES vol. 878, 1999, pages 598 - 602, XP008074011
- FURUZAWA-CARBALLEDA J. ET AL: 'Subcutaneous administration of polymerized-type I collagen for the treatment of patients with rheumatoid arthritis. An open-label pilo trial' THE JOURNAL OF RHEUMATOLOGY vol. 30, 2003, pages 256 - 259, XP008074019
- KROTZSCH-GOMEZ F.E. ET AL: 'Cytokine expression is downregulated by collagen-polyvinylpyrrolidone in hypertrophic scars' JOURNAL OF INVESTIGATIVE DERMATOLOGY vol. 11, 1998, pages 828 - 834, XP008074004

## Description

### Technical Field of the Invention

The present invention refers to a joint inflammation-modulating composition made of collagen-polyvinylpyrrolidone, it will be reviewed the incidence of joints inflammation in the Mexican population and the reasons by which the composition is considered immune-modulating as well as the studied parameters to consider the immune-modulating composition as such.

In consequence, the field of application is determined in the area of biological products applied in medicine.

### Background of the Invention

Rheumatoid arthritis (RA) is an autoimmune disease with a high prevalence in Mexican population (0.3%; range 0.1-0.6). RA is characterized by the presence of chronic inflammatory infiltrates, synovial hyperplasia, fibrosis and eventually joint cartilage degradation and subchondral bone erosion. The pathology is product of the variety and amount of activated cells that infiltrate synovial tissue, of the direct cell-cell contacts through cell adhesion molecules (CAMs) and of the pro-inflammatory cytokine overexpression that produce an imbalance towards an excessive joint tissue components degradation, related to destructive characteristics of the disease (Feldmann M, Brennan FM, Maini RN. Role of cytokines in rheumatoid arthritis. Ann Rev Immunol 1996;14:397-9).

Immunologically, RA synovial tissue is characterized by pro-inflammatory cytokine overproduction including interleukin-1β (IL-1β), tumor necrosis factor-α (TNF-α), that acts in a synergistically way and they are considered as "arthritogenic cytokines" (Cooper WO, Fava RA, Gates CA, et al. Acceleration of onset of collagen-induced arthritis by intra-articular injection of tumour necrosis factor or transforming growth factor-beta. Clin Exp Immunol 1992;89:244-50), IL-6, IL-8, IL-10, IL-15, IL-17, IL-18, transforming growth factor-β1 (TGF-β1), platelet derived growth factor (PDGF) and granulocyte monocyte-colony stimulating factor (GM-CSF) (Gowen M, Wood DD, Ihrie EJ, McGuire MKB, Russell RG. An interleukin-1 like factor stimulates bone resorption in vitro. Nature 1983;306:378-81*;* Moreland LW. Inhibitors of tumor necrosis factor for rheumatoid arthritis. J Rheumatol 1999; 26Suppl57:7-15; Manicourt DH, Triki R, Fukuda K, et al. Levels of circulating tumor necrosis factor-□ and interleukin-6 in patients with rheumatoid arthritis. Arthritis Rheum 1993;36:490-9*;* Dolhain RJEM, Tak PP, Brennan FM, Chantry D, Turner M, et al. Inhibitory effect of TNF-□ antibodies on synovial cell interleukin-1 production in rheumatoid arthritis. Lancet 1989;2:244-7*;* Brennan FM, Maini RN, Feldmann M Cytokine expression in chronic inflammatory disease. Br Med Bull 1995;51:368-84*;* Hildner K, Finotto S, Becker C, et al. Tumour necrosis factor (TNF) production by T cell receptor-primed T lymphocytes is a target for low dose methotrexate in rheumatoid arthritis. Clin Exp Immunol 1999;118:137-46*;* Minghetti PP, Blackburn WD Jr. Effects of sulfasalazine and its metabolites on steady state messenger RNA concentrations for inflammatory cytokines, matrix metalloproteinases, and tissue inhibitors of metalloproteinase, in rheumatoid synovial fibroblasts. J Rheumatol 2000; 27: 653-60*;* Furuzawa-Carballeda J, Alcocer-Varela J IL-8, IL-10, ICAM-1 and VCAM-1 expression levels are higher in synovial tissue from patients with rheumatoid arthritis than in osteoarthritis. Scand J Immunol 1999;50:215-22*).*

Arthritogenic cytokines have been detected not only in RA synovial fluid but also in synovial tissue *(*Gowen M, Wood DD, Ihrie EJ, McGuire MKB, Russell RG. An interleukin-1 like factor stimulates bone resorption in vitro. Nature 1983;306:378-81*;* Beutler BA. The role of tumor necrosis factor in health and disease. J Rheumatol 1999;26Suppl57:16-21*;* Black RA, Rauch ChT, Kozlosky CJ. A metalloproteinase disintegrin that releases tumour-necrosis factor- □ from cells. Nature 1997;385:729-33*;* Moreland LW. Inhibitors of tumor necrosis factor for rheumatoid arthritis. J Rheumatol 1999; 26Suppl57:7-15*;* Wakisaka S, Suzuki N, Takeba Y, et al. Modulation by proinflammatory cytokines of Fas/Fas ligand-mediated apoptotic cell death of synovial cells in patients with rheumatoid arthritis (RA). Clin Exp Immunol 1998;114:119-28*;* Kiener HP, Hofbauer R, Tohidast-Adrad M, et al. Tumor necrosis factor □ promotes the expression of stem cell factor in synovial fibroblasts and their capacity to induce mast cell chemotaxis. Arthritis Rheum 2000;43:164-74*;* Manicourt DH, Triki R, Fukuda K, et al. Levels of circulating tumor necrosis factor-□ and interleukin-6 in patients with rheumatoid arthritis. Arthritis Rheum 1993;36:490-9*;* Dolhain RJEM, Tak PP, Dijkmans BAC, et al. Methotrexate reduces inflammatory cell numbers, expression of monokines and of adhesion molecules in synovial tissue of patients with rheumatoid arthritis. Br J Rheumatol 1998;37:502-8*;* Manicourt D-H, Poilvache P, Van Egeren A, et al. Synovial fluid levels of tumor necrosis factor □ and oncostatin M correlate with levels of markers of the degradation of crosslinked collagen and cartilage aggrecan in rheumatoid arthritis but not in osteoarthritis. Arthritis Rheum 2000;43:281-8*;* Brennan FM, Chantry D, Turner M, et al. Inhibitory effect of TNF-□ antibodies on synovial cell interleukin-1 production in rheumatoid arthritis. Lancet 1989;2:244-7*;* Brennan FM, Maini RN, Feldmann M Cytokine expression in chronic inflammatory disease. Br Med Bull 1995;51:368-84*;* Hildner K, Finotto S, Becker C, et al. Tumour necrosis factor (TNF) production by T cell receptor-primed T lymphocytes is a target for low dose methotrexate in rheumatoid arthritis. Clin Exp Immunol 1999; 118:137-46*;* Minghetti PP, Blackburn WD Jr. Effects of sulfasalazine and its metabolites on steady state messenger RNA concentrations for inflammatory cytokines, matrix metalloproteinases, and tissue inhibitors of metalloproteinase, in rheumatoid synovial fibroblasts. J Rheumatol 2000; 27: 653-60*;* Furuzawa-Carballeda J, Alcocer-Varela J. IL-8, IL-10, ICAM-1 and VCAM-1 expression levels are higher in synovial tissue from patients with rheumatoid arthritis than in osteoarthritis. Scand J Immunol 1999;50:215-22*;* Dolhain RJEM, Tak PP, Dijkmans BAC, et al. Methotrexate reduces inflammatory cell numbers, expression of monokines and of adhesion molecules in synovial tissue of patients with rheumatoid arthritis. Br J Rheumatol 1998;37:502-8).

On the other hand, anti-inflammatory lymphokines such as IL-2, IL-4 and IFN-γ are not detected in the joint *(*Firestein GS, Xu W-D, Townsend K, et al. Cytokines in chronic inflammatory arthritis. I. Failure to detect T cell lymphokines (interleukin 2 and interleukin 3) and presence of macrophage colony-stimulating factor (CSF-1) and a novel mast cell growth factor in rheumatoid synovitis. J Exp Med 1988;168:1573-86*;* Salmon M, Gaston JSH. The role of T-lymphocytes in rheumatoid arthritis. Br Med Bulletin 1995;51:332-45*).*

Pro-inflammatory cytokines regulate in turn, the mechanisms of injury associated to the inflammatory focus and of pannus formation due to cytokines exert a direct effect in the specific receptors on the surface cell membrane expression. Amongst these receptors the selectin family *(*Chapman PT, Haskard DO. Leukocyte adhesion molecules. Br Med Bull 1995; 51:296-311), the members of the integrin family: *β1 subfamily* or VLA or Very Late Activation Antigens, β₂ *subfamily* or leukointegrins and *β₃ subfamily (*El-Gabalawy H, Canvin J, Gouping M, et al. Synovial distribution of □d/CD18, a novel leukointegrin. Arthritis Rheum 1996;39:1913-21*)* or cytoadhesin family as well as the immunoglobulin gene superfamily are including *(*Chapman PT, Haskard DO. Leukocyte adhesion molecules. Br Med Bull 1995; 51:296-311).

Immunohistochemical assayed to RA type B synoviocytes or synovial tissue, have shown alterations in the expression of some adhesion molecules: from the selectin family an increase of ELAM-1 and PEXCAM has been demonstrated. They are up-regulated by arthritogenic cytokines *(*Dolhain RJEM, Tak PP, Dijkmans BAC, et al. Methotrexate reduces inflammatory cell numbers, expression of monokines and of adhesion molecules in synovial tissue of patients with rheumatoid arthritis. Br J Rheumatol 1998;37:502-8*;* Chapman PT, Haskard DO. Leukocyte adhesion molecules. Br Med Bull 1995; 51: 296-311*;* El-Gabalawy H, Canvin J, Gouping M, et al. Synovial distribution of □d/CD18, a novel leukointegrin. Arthritis Rheum 1996;39:1913-21*;* Youssef PP, Triantafillou S, Parker A, et al. Variability in cytokine and cell adhesion molecule staining in arthroscopic synovial biopsies: quantification using color video image analysis. J Rheumatol 1997; 24: 2291-8*).*

From the integrin family a decrease of α₆β₁ has been determined, probably due to overexpression of IL-1β and TNF-α; αᵥβ₅ integrin has not expressed and α₁₋₅β₁ integrin and α_{L}, α_{M,} α_{X} and α_{d} leukointegrins have been increased related to normal synovial tissue *(*El-Gabalawy H, Canvin J, Gouping M, et al. Synovial distribution of □d/CD18, a novel leukointegrin. Arthritis Rheum 1996;39:1913-21*;* Youssef PP, Triantafillou S, Parker A, et al. Variability in cytokine and cell adhesion molecule staining in arthroscopic synovial biopsies: quantification using color video image analysis. J Rheumatol 1997;24:2291-8*;* Pirila L, Heino J. Altered integrin expression in rheumatoid synovial lining type B cells: In vitro cytokine regulation of □1□1, □6□1, and □v□5 integrins. J Rheumatol 1996;23:1691-8*;* Cicuttini FM, Martin M, Boyd AW. Cytokine induction of adhesion molecules on synovial type B cells. J Rheumatol 1994;21:406-12*).*

From the Immunoglobulin superfamily an increase in VCAM-1 and ICAM-1 expression has been demonstrated. The increased cell adhesion molecule expression is regulated by pro-inflammatory cytokines *(*Cicuttini FM, Martin M, Boyd AW. Cytokine induction of adhesion molecules on synovial type B cells. J Rheumatol 1994; 21: 406-12*).*

Likewise, in the RA physiopathology, calcium-independent and -dependent proteases (MMPs) play an important role in destructive joint phenomena and in pannus formation. Between calcium-independent proteases determined in high concentrations in rheumatoid synovium are cistein-proteases (cathepsin L), aspartic acid proteases (cathepsin D) and neutrophil elastase. Meanwhile MMP-1, -2, -3 and 13 but not MMP-9 and -10 are overexpressed in synovial tissue *(*Keyszer GM, Heer AH, Kriegsmann J, et al. Comparative analysis of cathepsin L, cathepsin D, and collagenase messenger RNA expression in synovial tissues of patients with rheumatoid arthritis and osteoarthritis, by in situ hybridization. Arthritis Rheum 1995;38:976-84*;* Owen CA, Campbell MA, Boukedes SS, Campbell EJ. Inducible binding of bioactive cathepsin G to the cell surface of neutrophils. J Immunol 1995;155:5803-10*;* Koolwijk P, Miltenburg AMM, Van Erck MGM, et al. Activated gelatinase-B (MMP-9) and urokinase-type plasminogen activator in synovial fluids of patients with arthritis. Correlation with clinical and experimental variables of inflammation. J Rheumatol 1995;22:385-93*;* Maeda S, Sawai T, Uzuki M, et al. Determination of interstitial collagenase (MMP-1) in patients with rheumatoid arthritis. Ann Rheum Dis 1995;54:970-5*;* Hembry RM, Bagga MR, Reynolds JJ, Hamblen DL. Immunolocalisation studies on six matrix metalloproteinases and their inhibitors, TIMP-1 and TIMP-2, in synovia from patients with osteo- and rheumatoid arthritis. Ann Rheum Dis 1995;54:25-32*;* Tetlow LC, Woolley DE. Mast cells, cytokines, and metalloproteinases at the rheumatoid lesion: dual immunolocalisation studies. Ann Rheum Dis 1995;54:896-903*).*

Assays performed in synovial fluid and serum from RA patients have been shown that mainly MMP-3, -1 and -9 are found in important amounts *versus* synovial fluid and serum obtained from controls. Protease concentration can be directly correlating with synovial inflammation intensity *(*Ishiguro N, Ito T, Obata K-I, et al. Determination of stromelysin-1, and 92 kDa type IV collagenase, Tissue Inhibitor of Metalloproteinase-1 (TIMP-1), and TIMP-2 in synovial fluid and serum from patients with rheumatoid arthritis. J Rheumatol 1996;23:1599-604*;* Watanabe Y, Shimamori N, Yamaguchi R, et al. Correlation between the appearance of gelatinases in the synovial fluid of patients with rheumatoid arthritis and polymorphonuclear elastase, stromelysin-1, and the tissue inhibitor of metalloproteinase-1. Clin Exp Rheumatol 1997;15:255-61*).*

Protease expression is up-regulated by pro-inflammatory cytokines, such as IL-1β and TNFα*(*Shingu M, Miyauchi S, Nagai Y, et al. The role of IL-4 and IL-6 in IL-1-dependent cartilage matrix degradation. Br J Rheumatol 1995; 34:101-6*;* West-Mays JA, Strissel KJ, Sadow PM, Fini ME. Competence for collagenase gene expression by tissue fibroblasts requires activation of an interleukin 1β autocrine loop. Proc Natl Acad Sci 1995;92:6768-72*;* Migita K, Eguchi K, Kawabe Y, et al. TNF-α-mediated expression of membrane-type matrix metalloproteinase in rheumatoid synovial fibroblasts. Immunol 1996;89:553-7*;* Cawston TE, Curry VA, Summers CA, et al. The role of oncostatin M in animal and human connective tissue collagen turnover and its localization within the rheumatoid joint. Arthritis Rheum 1998;41:1760-9*)* as well as for the integrins interaction with their ligand e.g. αᵥ or α₄ with fibronectin, β₂-microglobulin with major histocompatibility complex, VCAM- 1 with VLA-4, etc. *(*Larjava H, Lyons J, Salo R, et al. Anti-integrin antibodies induce type IV collagenase gene expression in keratinocytes. J Cell Physiol 1993;157:190-200*;* Romanic AM, Madri JA. The induction of 72 kD gelatinase in T cells upon adhesion to endothelial cells is VCAM-1 dependent. J Cell Biol 1994;125:1165-78*;* Tremble P, Chiquet-Ehrismann R, Werb Z. The extracellular matrix ligands fibronectin and tenascin collaborate in regulating collagenase gene expression in fibroblasts. Mol Biol Cell 1994;5:4390-3*;* Rükonen T, Westermarck J, Koivisto L, et al. Integrin □2□1 is a positive regulator of collagenase (MMP-1) and collagen □1(I) gene expression. J Biol Chem 1995; 270:13548-52*;* Migita K, Eguchi K, Tominaga M, et al. □2-Microglobulin induces stromelysin production by human synovial fibroblasts. Biochem Biophys Res Comm 1997;239:621-5*;* Sarkissian V, Lafyatis R. Integrin engagement proliferation and collagenase expression of rheumatoid synovial fibroblasts. J Immunol 1999;162:1772-9*).* Inhibition in protease expression and/or activity is regulated by IL-4, IL-6, IL-10 and INF-*γ*(Prontera C, Crescenzi G, Rotilio D. Inhibition by interleukin-4 of stromelysin expression in human skin fibroblasts: role of PKC. Exp Cell Res 1996;224:183-8*;* Lacraz S, Nicod LP, Chicheportiche R, et al. IL-10 inhibits metalloproteinase and stimulates TIMP-1 production in human mononuclear phagocytes. J Clin Invest 1995;96:2304-10*;* Busiek DF, Baragi V, Nehring LC, et al. Matrilysin expression by human mononuclear phagocytes and its regulation by cytokines and hormones. J Immunol 1995;159: 6484-91*;* Overall ChM. Regulation of tissue inhibitor of matrix metalloproteinases. In: Inhibition of matrix metalloproteinases. Therapeutic potential. Editores: Greenwald RA, Golub LM 1994;732:59-64*)* or by non-specific inhibitors such as α₁-antitripsin or α₂-macrogrobulin and specific inhibitors such as tissue inhibitors of metalloproteinases (TIMPs) *(*Hayakawa T, Yamashita K, Tanzawa K, et al. Growth-promoting activity of tissue inhibitors of metalloproteinase-1 (TIMP-1) for a wide range of cells. A possible new growth factor in serum. FEBS Lett 1992;298:29-32*).* TIMPs are linked with high affinity, in a non-covalent and irreversible manner forming stechiometric complexes (1:1) with MMPs proforms and active forms *(*Bodden MK, Windsor LJ, Caterina NM, et al. Analysis of the TIMP-1/FIB-CL complex. In: Inhibition of matrix metalloproteinases. Therapeutic potential. Editors: Greenwald RA, Golub LM 1994;85-95*).* TIMP-1 and -3 are distributed through all RA synovial tissue. Nevertheless, TIMP-2 is not detected *(*Hembry RM, Bagga MR, Reynolds JJ, Hamblen DL. Immunolocalisation studies on six matrix metalloproteinases and their inhibitors, TIMP-1 and TIMP-2, in synovia from patients with osteo- and rheumatoid arthritis. Ann Rheum Dis 1995;54:25-32*).*

Osteoarthritis (OA) or degenerative joint disease is the most common rheumatic disorder. The prevalence of degenerative joint disease is similar for both genders as well as the radiological characteristics, with or without symptoms which are well known worldwide beyond 60 or 70 years. OA affects 2.3% of Mexican population (range 1.7-2.9). The World Health Organization and the American Academy of Orthopedic Surgery, in their proposal criteria for degenerative joint disease defined it as: "Osteoarthritis is the result both of mechanic and biologic events that destabilize the normal coupling of degradation and synthesis of joint cartilage and subchondral bone. Although it may be initiated by multiple factors including genetic, developmental, metabolic and traumatic; OA involves all of the tissues of the diarthrodial joint. Ultimately, OA is manifested by morphologic, biochemical, molecular, and biomechanical changes of both cells and matrix which leads to a softening, fibrillation, ulceration and loss of joint cartilage, sclerosis and eburnation of subchondral bone, osteophytes, and subchondral cysts. When clinically evident, OA is characterized by joint pain, tenderness, limitation of movement, crepitus, occasional effusion, and variable degrees of local inflammation" *(*Ramos NF. Enfermedad articular degenerativa. In: Enfermedades Reumáticas. Criterios y Diagnóstico. Editorial McGraw-Hill Interamericana. 1α. Edición, Tomo II; 1999, págs:437-63*;* Pelletier J-P, Martel-Pelletier J, Abramson SB. Osteoarthritis, an inflammatory disease. Arthritis Rheum 2001; 44:1237-47*).*

Clinically, OA is characterized by arthritis pain, pain over pressure, disability, crepitus, occasional increased of synovial fluid and several degrees of local inflammation without systemic disorders.

Pathologically OA is characterized by irregular cartilage damage, more frequently in areas supporting weight, subchondral bone sclerosis, subchondral cysts, marginal osteophytes, increase of metaphisiary blood influx and variable degrees of synovitis.

Histologically, OA is featured by early fragmentation of joint surface, chondrocytes aggregation, vertical cartilage breaking, and variable crystal deposition and lately line violation by blood vessels. It is also characterized by wound repair, specifically of the osteophytes and then by total cartilage damage, sclerosis and subchondral bone focal osteonecrosis.

Biomechanically, AO is featured by the alteration of tensile properties, compression and sliding as well as cartilage hydraulic permeability, water accumulation and exceeding volume. Cartilage changes are accompanied by increasing bone stiffness.

Biochemically, AO is characterized by decreasing proteoglicans concentration, possible alteration of proteoglicans size and aggregation, collagen size and texture alterations, increase turnover of matrix macromolecules *(*Lark MW, Bayne EK, Flanagan J, et al. Aggrecan degradation in human cartilage. Evidence for both matrix metalloproteinase and aggrecanase activiy in normal, osteoarthritic and rheumatoid joints. J Clin Invest 1997;100:93-106*;* Ramos NF. Enfermedad articular degenerativa. In Enfermedades Reumáticas. Criterios y Diagnóstico. Editorial McGraw-Hill Interamericana. 1a. Edición, Tomo II; 1999, págs:437-63*;* Rowan AD, Koshy PJT, Shingleton WD, et al. Synergistic effects of glycoprotein 130 binding cytokines in combination with interleukin-1 on cartilage collagen breakdown. Arthritis Rheum 44:1620-32*).*

*Therapeutically, OA is featured as lacking of a specific treatment (*Felson DT. The veredict favors nonsteroidal antiinflammatory drugs for treatment of osteoarthritis and a plea for more evidence on other treatments. Arthritis Rheum 2001;44:1477-80*).*

Immunologically, OA is associated with pro-inflammatory cytokines, adhesion molecule and protease overexpression, which is qualitatively similar to those detected in RA but quantitatively appear in lower concentration than in the rheumatic joint *(*Pelletier J-P, Martel-Pelletier J, Abramson SB. Osteoarthritis, an inflammatory disease. Arthritis Rheum 2001; 44:1237-47*;* Furuzawa-Carballeda J, Alcocer-Varela J. IL-8, IL-10, ICAM-1 and VCAM-1 expression levels are higher in synovial tissue from patients with rheumatoid arthritis than in osteoarthritis. Scan J Immunol 1999;50:215-22*;* Rowan AD, Koshy PJT, Shingleton WD, et al. Synergistic effects of glycoprotein 130 binding cytokines in combination with interleukin-1 on cartilage collagen breakdown. Arthritis Rheum 44:1620-32*;* Furuzawa-Carballeda J, Alcocer-Varela J. IL-8, IL-10, ICAM-1 and VCAM-1 expression levels are higher in synovial tissue from patients with rheumatoid arthritis than in osteoarthritis. Scan J Immunol 1999;50:215-22*;;* Cronstein BN, Weissman R. The adhesion molecules of inflammation. Arthritis Rheum 1993;36:147-57*;* Pelletier J-P, Martel-Pelletier J, Abramson SB. Osteoarthritis, an inflammatory disease. Arthritis Rheum 2001; 44:1237-47*;* Keyszer GM, Heer AH, Kriegsmann J, et al. Comparative analysis of cathepsin L, cathepsin D, and collagenase messenger RNA expression in synovial tissues of patients with rheumatoid arthritis and osteoarthritis, by in situ hybridization. Arthritis Rheum 1995;38:976-84*;* Okada Y, Naka K, Kawamura K, et al. Localization of matrix metalloproteinase 9 (92-kilodalton gelatinase/type IV collagenase = gelatinse B) in osteoclasts: Implications for bone resorption. Lab Invest 1995; 72:311-22*;* Keyszer GM, Heer AH, Kriegsmann J, et al. Comparative analysis of cathepsin L, cathepsin D, and collagenase messenger RNA expression in synovial tissues of patients with rheumatoid arthritis and osteoarthritis, by in situ hybridization. Arthritis Rheum 1995;38:976-84*;* Okada Y, Naka K, Kawamura K, et al. Localization of matrix metalloproteinase 9 (92-kilodalton gelatinase/type IV collagenase = gelatinse B) in osteoclasts: Implications for bone resorption. Lab Invest 1995; 72:311-22*;* Prontera C, Crescenzi G, Rotilio D. Inhibition by interleukin-4 stromelysin production by human synovial fibroblasts. Biochem Biophys Res Comm 1997; 239:621-5*;* Lacraz S, Nicod LP, Chicheportiche R, et al. IL-10 inhibits metalloproteinase and stimulates TIMP-1 production in human mononuclear phagocytes. J Clin Invest 1995;96:2304-10*;* Overall ChM. Regulation of tissue inhibitor of matrix metalloproteinases. In: Inhibition of matrix metalloproteinases. Therapeutic potential. Editores: Greenwald RA, Golub LM, 1994;732:59-64*;* Haywood L, McWilliams DF, Pearson CI, Gill SE, Ganesan A, Wilson D, Walsh DA. Inflammation and angiogenesis in osteoarthritis. Arthritis Rheum 2003;48:2173-7*).*

OA is a disease having an important effect on the socioeconomic aspect of population as occupying the first place between the ten more frequent causes of morbidity in persons aged 65 or more. To such a disease no specific treatment exists and the most important therapy is paracetamol, non-steroidal anti-inflammatories (NSAIDS) or both which are useful indeed to control symptoms, however they same do not modify natural evolution of the disease. Thus, with the aim to improve quality of life in the patient suffering OA and in view of the pharmacologic background of the PVP-collagen (negative control of some proinflammatory cytokines, adhesion molecules and proteases), it is suggested that the biodrug can assist in the induction of sustained remissions through the modulating effect of the inflammatory process producing no collateral or adverse effects, but improving the viscosity of the joint cavity so preventing cartilage degradation by biomechanical damage.

Current treatment of OA is targeted primarily at symptomatic relief. Non-pharmacologic approaches, such as weight loss, muscle-strengthening exercises, bracing, and joint protection have the potential for structure modification. Pharmacologic modalities including simple analgesics, non selective non-steroidal anti-inflammatory drugs (NSAIDS), cyclooxigenase-2 (Cox-2) specific inhibitors, intraarticular (IA) glucocorticoids are directed toward relief of pain and inflammation. IA hyaluronan (hyaluronate, hyaluronic acid) or high molecular weight hyaluronan injection (viscosupplementation) is another therapeutic approach for knee OA unresponsive to conventional measures in patients who wish to delay or avoid knee joint replacement surgery. Advances over the past several decades in the understanding of OA pathophysiology, provide a more rational opportunity to target pathways involved in the degenerative process^{2,3}.

Current treatment of OA is targeted primarily at symptomatic relief. Non-pharmacologic approaches, such as weight loss, muscle-strengthening exercises, bracing, and joint protection have the potential for structure modification. Pharmacologic modalities including simple analgesics, non selective non-steroidal anti-inflammatory drugs (NSAID), cyclooxigenase-2 (Cox-2) specific inhibitors, intraarticular (IA) glucocorticoids are directed toward relief of pain and inflammation. IA hyaluronan (hyaluronate, hyaluronic acid) or high molecular weight hyaluronan injection (viscosupplementation) is another therapeutic approach for knee OA unresponsive to conventional measures in patients who wish to delay or avoid knee joint replacement surgery. Advances over the past several decades in the understanding of OA pathophysiology, provide a more rational opportunity to target pathways involved in the degenerative process^{2,3}.

Among the multiple therapies employed for inflammation suppression and chronic rheumatoid synovitis, drugs such as NSAIDS modify prostaglandins, thromboxanes and prostacyclins, antimalarial drugs (hydroxichloroquine and chloroquine) stabilize lysosome membranes, cyclophosphamide together with alkylant agents, induce proliferating cell death. Azathioprine is a cytotoxic immunosuppresor with anti-inflammatory effects that inhibits NK and B cell function and IL-6 synthesis *(*Choy E, Kingsley G. How Do Second-Line Agents work?. Br Med Bulletin 1995;51:472-92*).* Low doses of methotrexate increases adenosine release by endothelial cells, neutrophils and damaged fibroblasts; by β₂ integrin adhesion inhibition and in consequence modify leukocyte migration. Also, methotrexate inhibits cell proliferation of dividing cells by thymidilate synthetase and carboxyamide ribonucleotide transformilase control. Corticoids, such as prednisone and dexamethasone, indirectly inhibit ICAM-1 and E-selectin expression in the activated endothelium, through a mechanism that involves inhibition of IL-1 synthesis, as well as other pro-inflammatory cytokines *(*Chapman PT, Haskard DO. Leukocyte adhesion molecules. Br Med Bull 1995; 51: 296-311*).*

Biological therapies include anti-CAMs antibodies directed to inhibit adhesion of leukocytes to endothelium, such as murine anti-ICAM-1 monoclonal antibody, that avoids inflammatory cell migration towards joint cavity. *(*Kavanaugh A, Nichols L, Davis L, Rothlein R, Lipsky P. Anti-CD54 (Intercellular Adhesion Molecule-1; ICAM-1) Monoclonal Antibody Therapy in Refractory Rheumatoid Arthritis. Arthritis Rheum 1993;49:48-51*).* Those therapies that avoid cytokine effects, i.e. neutralizing antibodies against IL-6, TGF-β1 or TNF-αPaleolog EM, Hunt M, Elliott MJ, Feldmann M, Maini RN, Woody JN. Deactivation of vascular endothelium by monoclonal anti-tumor necrosis factor □ antibody in rheumatoid arthritis. Arthritis Rheum 1996;39:1082-91*;* Brennan FM, Browne KA, Green PA, Jaspar J-M, Maini RN, Feldmann M Reduction of serum MMP-1 and MMP-3 in rheumatoid arthritis patients following anti-tumour necrosis factor-□ (cA2) therapy. Br J Rheumatol 1997; 36: 643-50*;* Perkins DJ, St Clair EW, Misukonis MA, Weinberg B. Reduction of NOS2 overexpression in rheumatoid arthritis patients treated with anti-tumor necrosis factor □ monoclonal antibody (cA2). Arthritis Rheum 1998;41:2205-10*;* Breedveld FC, Van del Lubbe P. Monoclonal Antibody Therapy of Inflammatory Rheumatic Diseases. Br Med Bulletin 1995;51:493-502*).*

Nevertheless, long term clinical results of those therapies have not been ideal, since the cost of the treatment, different adverse collateral effects and the poor pharmacokinetics (diminution on the timing for cA2 administration), generation of anti-globulins for cA2, as well as the redundant nature of other cytokines can affect the therapeutic value of those antibodies.

Biotherapies that diminish pro-inflammatory cytokine production, include vaccines anti-T cells, monoclonal antibodies anti-major histocompatibility complex-DR or anti-T cell accessory molecules, such as CD28, CD4, CD6, CD5, or IL-2R *(*Breedveld FC, Van del Lubbe P. Monoclonal Antibody Therapy of Inflammatory Rheumatic Diseases. Br Med Bulletin 1995;51:493-502*).*

Even those biotherapies that produce peripheral immune tolerance, through oral administration of proteins, mainly type II collagen isolated from non-lathyritic sternal chicken cartilage or from shark cartilage and solubilized in acetic acid 0.1 M, during 3 months, every morning and 20 minutes before any meal consumption *(*Thompson HSG, Staines NA. Gastric Administration of Type II Collagen Delays the Onset and Severity of Collagen-Induced Arthritis in Rats. Clin Exp Immunol 1985;64:581-6*;* Trentham DE, Dynesius-Trentham RA, Orav EJ, Combitchi D, Lorenzo C, Sewell KL, Hafler DA, Weiner HL. Effects of Oral Administration of Type II Collagen on Rheumatoid Arthritis. Science 1993; 261:1727-30*)* or type I and III collagens *(*Zhang ZJ, Lee ChSY, Lider O, Weiner HL. Suppression of Adjuvant Arthritis in Lewis Rats by Oral Administration of Type II Collagen. J Immunol 1990;145:2489-94*),* where these proteins work through autoreactive CD4+ cells anergy by a shared epitope present in the three type of collagens or by a restriction of the type I or II main histocompatibility complex from the activated joint cells, favoring cytokine release that inactivate autoreactive cells *(*Thompson HSG, Staines NA. Gastric Administration of Type II Collagen Delays the Onset and Severity of Collagen-Induced Arthritis in Rats. Clin Exp Immunol 1985;64:581-6*; Trentham DE,* Dynesius-Trentham RA, Orav EJ, Combitchi D, Lorenzo C, Sewell KL, Hafler DA, Weiner HL. Effects of Oral Administration of Type II Collagen on Rheumatoid Arthritis. Science 1993; 261:1727-30*;* Zhang ZJ, Lee ChSY, Lider O, Weiner HL. Suppression of Adjuvant Arthritis in Lewis Rats by Oral Administration of Type II Collagen. J Immunol 1990;145: 2489-94*;* Barinaga M Treating Arthritis With Tolerance. Science 1993; 261:1669-70*).*

Finally, joint replacement surgery by prosthesis or another different treatment involving plasmapheresis, leukopheresis or total lymphoid irradiation.

### DETAILED DESCRIPTION FOR THE INVENTION

The present invention is directed to the use of a collagen-polyvinylpyrrolidone biocomplex for preparing a drug for the treatment of osteoarthritis.

Due to drugs mentioned above, immunotherapy, biological therapy or alternatives not only have non-satisfactory results but also produce adverse effects or important secondary side effects. For this reason, we suggest to evaluate the biodrug, collagen-polyvinylpyrrolidone (Fibroquel®, Reg. No. 210M95 SSA) in RA or OA patients. Based on the fact that the addition of extracellular matrix proteins to T lymphocyte cultures is capable of modulating T cell response *(*Easter DW, Hayt DB, Ozkan AN. Immunosuppression by a peptide from the gelatin binding domain of human fibronectin. J Surg Res 1988;45:370-5*;* Rybski JA, Lause DB, Reese AC. Effect of fibronectin on antigen-induced lymphoproliferation and antibody synthesis in rats. J Leuk Biol 1989;45:35-45*;* Rüeg CR, Chiquet-Ehrismann R, Alkan SS. Tenascin, an extracellular matrix protein, exerts immunomodulatory activities. Proc Natl Acad Sci USA 1989;86:7437-41*;* Furuzawa-Carballeda J, Alcocer-Varela J, Diaz de León L. Collagen-PVP decreases collagen turnover in synovial tissue cultures from rheumatoid arthritis patients. Ann NY Acad Sci 1999;878:508-602*); that oral administration of type II collagen (*Thompson HSG, Staines NA. Gastric Administration of Type II Collagen Delays the Onset and Severity of Collagen-Induced Arthritis in Rats. Clin Exp Immunol 1985;64:581-6*;* Trentham DE, Dynesius-Trentham RA, Orav EJ, Combitchi D, Lorenzo C, Sewell KL, Hafler DA, Weiner HL. Effects of Oral Administration of Type II Collagen on Rheumatoid Arthritis. Science 1993; 261:1727-30*)* or type I or III collagen *(*Zhang ZJ, Lee ChSY, Lider O, Weiner HL. Suppression of Adjuvant Arthritis in Lewis Rats by Oral Administration of Type II Collagen. J Immunol 1990;145: 2489-94*)* modulates peripheral immune system in RA and also that collagen-PVP has anti-inflammatory properties, as mention below.

Collagen-PVP is a γ-irradiated mixture of pepsinized porcine type I collagen and PVP in a citric buffer, that stabilizes pH. In culture medium at 37°C and neutral pH, collagen-PVP does not form a gel like other collagens and its electrophoretic, physicochemical and pharmacological properties are modified by the covalent binding between protein and PVP. It has been demonstrated *in vitro* and *in vivo* that the components alone (collagen and PVP) do not have the same properties than collagen-PVP *(*Chimal-Monroy J, Bravo-Ruiz T, Krötzsch-Gómez FE, Díaz de León L. Implantes de Fibroquel aceleran la formación de hueso nuevo en defectos óseos inducidos experimentalmente en cráneos de rata: un estudio histológico. Rev Biomed 1997;8:81-8*).* This biodrug has demonstrated to modulate collagen metabolism, pro-inflammatory cytokine expression, to improve skin wound repair, as well as other tissues.

Previous data have been determined by experiments performed in Wistar rats and humans, where intralesional treatment of collagen-PVP of surgical wounds stimulates an increase of the granulation tissue on day 7 after surgery and tissues exhibit better skin architecture at day 28, when compared to non treated group. Improvement include cutaneous appendages presence in the wounded zone (sebaceous glands and hair follicles) together with a normal skin like collagen bundle arrangement *(*Krötzsch-Gómez FE, Guerrero-Padilla E, Diaz de León L. Morphological studies on the effects of Fibroquel during wound of surgical wounds in rats. J Cell Biochem 1993; Suppl, 17E:137R506*).*

Bone repair, preclinical and clinical assessment.

### Fracture model in rats.

Femoral diaphisary fractures were performed in Wistar rats, after that 0.2 ml of collagen-PVP were administered in situ during the 3 first days after lesion. Bone consolidation parameters were evaluated and 67% of the treated animals showed total bone repair at day 30 *vs.* only 20% for the control group. Histological analysis demonstrated an early turnover of the mesenchymal cells in the collagen-PVP treated group by cartilage tissue first, and later by osteoblasts and trabecular bone (days 16 and 23). Also, it was determined higher osteopontin and osteonectin expression, meanwhile type I collagen and fibronectina were similar in experimental and control groups. Results suggest that collagen-PVP modulates mesenchymal cell growth factors and extracellular matrix protein expression, stimulating migration, proliferation and differentiation of chondrogenic and osteogenic cells, improving bone repair *(*Almazán Diaz A, de la Cruz Garcia JC, Lira Romero JM, Arrellin G, Chimal Monroy J, Diaz de León L, Furuzawa- Carballeda J, Krötzsch Gómez F: Investigación experimental de la regeneración ósea en fémures de rata después de la aplicación de colágena I polimerizada: Estudio radiológico, histológico e inmunohistoquimico. Rev Mex Ortop Traum 1996;10:142-52*;* Chimal-Monroy J, Bravo-Ruiz T, Furuzawa-Carballeda GJ. Lira RJM, De la Cruz GJC, Almazán DA, Krötzsch-Gómez FE, Arrellin RG, Diaz de León L. Collagen-PVP accelerates new bone formation of experimentally induced bone defects in rat skull and promotes the expression of osteopontin and SPARC during bone repair of rat femora fractures. Ann NYAcad Sci 1998;857:232-6*).*

### Bone repair in humans.

Also, collagen-PVP effect was evaluated in diaphisary fractures of tibia in a phase I clinical trial in 20 patients (10 experimental and 10 placebo). The study included early closed fractures and all of them were stabilized in the first 7 days. Collagen-PVP or placebo (0.2 ml) were administered intralesionally during surgery at days 1, 7 and 30, through the surgical wound. At day 30, 80% of the patients treated with collagen-PVP reported pain absence and the other 20% mild pain. In contrast, 50% of the control group patients exhibited moderate pain and the other 50% mild pain. Also, collagen-PVP treated group did not show inflammatory signs in the damaged area, but 50% of the control group showed it. Besides, serum alkaline phosphatase was increased in the collagen-PVP treated group, when compared with controls.

At day 30, radiographic bone repair according to Meller scale, was grade I and II in the 100% of collagen-PVP treated patients at days 30 and 90, respectively; different to controls where they were 50% and 100% grade I at days 30 and 90, respectively.

Bone repair of the collagen-PVP treated group was appropriate, all patients tolerated bipedal displacement, and there were not knee or ankle movement limitation. All these patients exhibited progressive walk before week 12, and none of them required specific rehabilitation. Control group also exhibited bone repair, although they did not tolerate bipedal displacement without external support at the same 12 week. Four of them required specific rehabilitation due to muscle atrophy.

In summary, collagen-PVP accelerate bone repair process by up regulation of osteopontine, osteonectin and alkaline phosphatase, whom favors differentiation to the mesenchymal tissue towards cartilage, hypertrophic cartilage later and finally to bone tissue. Perhaps that regulation was due to down modulation of pro-inflammatory cytokine expression (mainly IL-1 y TNF-α), adhesion molecules (ELAM-1, VCAM-1 e ICAM-1) and some proteinases and in consequence early pain control because prostaglandin synthesis regulation *(De la Cruz Garcia JC. Efecto de la colágena tipo I polimerizada al 1% inyectable en la consolidación ósea (reporte preliminar). Tesis. Hospital de Traumatologia y Ortopedia de "Lomas Verdes". UNAM 1997).*

### Collagen-PVP effect on tibia pseudoarthrosis.

Patients with tibia pseudoarthrosis aged 6 to 72 years old received 1 ml of collagen-PVP or placebo during 6 weeks. After treatment, from the collagen-PVP treated group 16 patients (51.6%) exhibited excellent bone repair (grade III, according to Meller scale), in 12 patients (38.7%) was good (grade II) and only in 3 of them (9.68%) was not appropriate (grade I). Meanwhile in the placebo treated group no bone repair was observed in any of the treated patients. Histological analysis showed appropriate osteoblastic activity in the collagen-PVP treated group. Besides pain was diminished in patients treated with the copolymer, may be through inflammatory factor modulation, via Cox (prostaglandin production),and in consequence patients showed strength and appropriate muscle tone in the affected limb *(*Bermúdez-Hickey R, Nésme-Ávila W, Ruiz-Flores L, Suárez E. Tratamiento de la pseudoartrosis de tibia con colágeno-polivinilpirrolidona. Rev Mex Ortop Traum 1999;13:148-51*).*

Clinical experience in dermal fibrotic diseases associated to chronic inflammation.

Collagen-PVP treatment of hypertrophic scarring or morphea.

Intralesional injection of biodrug once per week during 1 to 3 months in human hypertrophic scars or scleroderma lesions diminishes pruritus, pain, erythema, volume, and inflammatory infiltrates. It makes that tissue architecture resembles normal skin. Moreover, the biodrug modulates ECM turnover, mainly types I and III collagen, and down-regulates the expression level of IL-1β, TNF-α, PDGF and VCAM-1 *(*Krötzsch-Gómez FE, Furuzawa-Carballeda J, Reyes-Márquez R, Quiróz-Hernández E and Diaz de León L. Cytokine expression is downregulated by collagen-polyvinylpyrrolidone in hypertrophic scars. J Invest Dermatol, 1998; 111:828-834*.*

Furuzawa-Carballeda J, Krötzsch E, Espinosa-Morales R, Alcalá M, Barile-Fabris L. Subcutaneous administration of collagen-polyvinylpyrrolidone down-regulates IL-1□, TNF-□, TGF-□1, ELAM-1and VCAM-1 expression in scleroderma skin lesions. Clinical and Experimental Dermatology. 2005; 30:83-86*).*

### Collagen-P VP effect for the treatment of scleroderma skin lesions.

Collagen-PVP immunomodulating effect was also observed in scleroderma skin lesions, due to treated lesions at the same posology prescribed above during 3 months, improved texture and appearance skin. Histologically, Collagen-PVP-treatment recovered cutaneous appendages, type III collagen in papillary dermis and type I/III collagen proportion, whilst thick bundles of type I collagen decreased. It was also determined by immunohistochemistry that, collagen-PVP down-regulated IL-1β and ELAM-1 expression *(*Barile L, Furuzawa-Carballeda J, Krötzsch-Gómez FE, Espinosa-Morales R, Alcalá M, Diaz de León L. Comparative study of collagen-polyvinylpyrrolidone vs. triamcinolone acetate in systemic sclerosis. Clin Exp Rheumatol 1998;16:370*).*

Based on the results that collagen-PVP down-regulates some proinflammatory cytokines, adhesion molecules and collagen turnover in skin fibrotic disorders, associated with chronic inflammation, we suspected that the biodrug could have the same effect on inflamed synovial tissue cultures from RA patients.

### Safety studies

### Lymphoproliferation studies.

Assays showed a marked proliferation of purified lymphocytes when cultured in plates with phytohaemaglutinin (PHA) or with PHA and collagen-PVP in serum-free culture system. Moreover, no proliferation above baseline was observed when cells were cultures with collagen-PVP alone. These results suggest that the biodrug was not able to stimulate lymphocyte proliferation.

### Geno- and mielotoxicity studies.

The SCGE assay or "comet assay" was carried out with a cell suspension of lymphocytes embedded in an agar gel sandwich on a microscope slide, lysed and electrophoresed for a short time under alkaline conditions. Lysis removes cell contents except for the nuclear material and

DNA remains highly supercoiled in the presence of a small amount of non-histone protein; when placed in alkali, it starts to unwind from sites of strand breakage. Cells with increased DNA damage display increased migration of DNA from the nucleus towards the anode under an electrical current, giving the appearance of a "comet tail". In this work, the analysis of DNA damage has demonstrated that cultures incubated with or without collagen-PVP did not induce any change in DNA migration in lymphoid cell cultures. Therefore, these data suggest that the exposure to collagen-PVP did not have a genotoxic effect.

### Laboratory studies

Safety, follow-up, clinical evaluation and laboratory tests have been performed in healthy volunteers and patients with hypertrophic scars or scleroderma. Hematological tests (white blood cells index, blood chemistry, serum calcium levels), urinalysis, liver function test did not show differences before and after collagen-PVP administration. These findings indicate that the treatment did not induce any adverse immediate clinical reaction.

### Anti-porcine collagen or anti-collagen-PVP antibodies

Total serum samples were tested by means of ELISA for reactivity against porcine type I collagen or collagen-PVP. Serum samples from 110 healthy volunteers no treated with the biodrug were used as a negative control to establish baseline levels of reactivity. Serum samples from 37 SSc patients were used as a positive control, because previous reports have identified circulating human anti-collagen antibodies in several patients. Serum samples from 44 individuals treated with collagen-PVP, were used to examine serologic reactivity to collagen-PVP, these samples were plotted according to the number of collagen-PVP doses (less than 5, 6 to 10, 11 to 20, 21 to 100 and more than 100 administrations). Also, serum sample reactivity was compared using an average group analysis according to Dunnett test in at least two different dilutions. A significant difference was observed by the presence of antibodies against porcine type I collagen between healthy donors and SSc patients (p<0.05); no positive correlation could be determined on antibodies against collagen-PVP (even considering factors such as the dosage, number of doses or patient age) *(*Furuzawa-Carballeda J, Castillo I, Rojas E, Valverde M, Diaz de León E, Krötzsch E. Cellular and humoral responses to collagen-polyvinylpyrrolidone administered during short and long periods in humans. Can J Pharmacol Physiol 2003;81.1029-35).

On the other hand, the safety of the polymer, PVP, is useful in many pharmaceutical formulations, foods, cosmetics and toiletries as well as industrial applications, due to its particular physical and chemical properties (the homopolymer of N-vinyl-2-pyrrolidone) is a biologically inert and safe polymer. Many studies have been shown its innocuity. PVP used in collagen-PVP is low molecular weight (Robinson *et al.* 1990).

PVP used to manufacture collagen-PVP is of low molecular weight material due to it is excreted by the kidney. There are no reported adverse effects following intravenously administration as a plasma expansor, or of high doses orally, subcutaneous or intramuscular post-administration *(*Robinson BV, Sullivan FM, Bazelleca JF, Schwartz SL. PVP. A critical review of the kinetics and toxicology of polyvinylpyrrolidone (Povidone). Lewis Publishers, Inc. 1990*).*

### Preclinical assays in synovial tissue cultures from rheumatoid arthritis (RA) patients (no part of the invention)

*Collagen-PVP effect in synovial tissue cultures from RA patients.* To evaluate whether collagen-PVP produces an effect either on cell metabolism or on cell concentration, we measured the DNA content. There were no statistically significant differences in DNA content when treated and control cultures were compared (Table 1). It is important to mention that DNA content in non-RA synovium was 8 to 10-fold lower than that determined in RA synovial tissue. There were no differences between control and collagen-PVP-treated cultures.

**Table 1. Collagen-PVP effect on DNA in synovial tissue cultures from non-RA and RA patients.**

| | Treatment | - | - | - | + | + | + |
|---|---|---|---|---|---|---|---|
| | Culture day | 3^{rd} | 5^{th} | 7^{th} | 3^{rd} | 5^{th} | 7^{th} |
| Sample (mean ± SD)* | | | | | | | |
| Non- RA | | 0.50±0.08 | 0.47±0.08 | 0.46±0.08 | 0.40±0.07 | 0.51±0.08 | 0.45±0.08 |
| RA | | 4.27±1.20 | 3.92±1.11 | 5.45±1.68 | 4.69±1.48 | 4.71±1.64 | 3.84±1.21 |

The results depict mean ± SD of DNA content (µg/ml) in synovial tissue cultures from 5 non-RA and 10 RA patients each sample done in quadruplicate synovial tissue homogenates. (-) without collagen-PVP; (+) with 1% collagen-PVP.

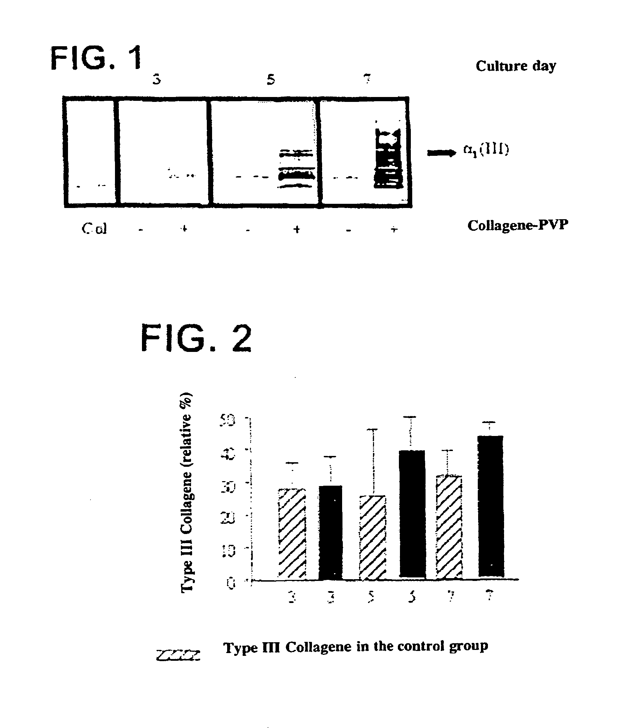

*Histological findings in synovial tissue cultures from non-RA and RA patients.* Morphological evaluation of non-RA synovium cultured with 1% collagen-PVP showed unaltered tissue architecture on the 3^{rd} and 7^{th} culture day (Fig. 1*A*). The addition of collagen-PVP to non-RA synovium cultures did not modify tissue architecture, fiber collagen thickness or type I/III collagen proportion (Fig. 1*B*). However, histological evaluation of synovial tissue from RA control cultures showed a variable content of inflammatory cells and fibrosis with abundant type I collagen (red fibers in Fig. 1*C*) at 3^{rd}, and 7^{th} culture day. In contrast, paired 1% collagen-PVP-treated cultures showed recovery of type III collagen (blue fibers in Fig. 1*D*, at 7^{th} culture day), similar to normal synovial tissue.

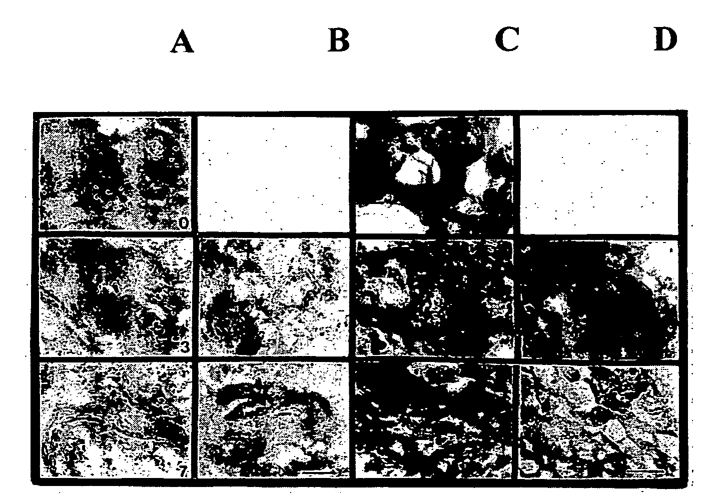

**Fig. 1****.** Collagen-PVP effect on tissue architecture of synovium in culture. Photomicrographs of synovial tissue cultures with or without 1% collagen-PVP, stained with the Herovici technique. (A) Non-RA synovial control cultures at 0, 3^{rd} and 7^{th} culture day. (B) Non-RA synovium collagen-PVP-treated cultures during 3 and 7 days. (C) RA synovial tissue incubated without treatment during 0, 3 and 7 days, respectively. The predominant extracellular matrix component is type I collagen (fibers in red). (D) RA synovium treated with collagen-PVP during 3 and 7 days, respectively. There is an increase of type III collagen (fibers in blue). Scale bar: 100 µm. *Relative percentage of types I and III collagen in synovial tissue cultures from non-RA and RA patients.* In order to confirm the change in the relative proportions of types I and III collagen, duplicates of synovium homogenates at each point of culture were evaluated by interrupted gel electrophoresis and densitometric analysis. Under reducing conditions, the inter-chain disulfide bonds are cleaved, releasingα₁(III) monomers of collagen, which migrate more slowly than □₁(1) chains of type I collagen. Fig. 2*A* shows the fine band of α₁(III) in the synovial tissue control cultures. Densitometric analysis showed that the addition of collagen-PVP to non-RA synovium cultures did not produce any modification in the relative percentage of type I or type III collagen (Fig. 2*B*). However, RA synovium cultures treated at 7^{th} day displayed a 1.7 fold increase in the α₁(III) band, in a time-dependent fashion (p<0.009, treated *vs.* untreated cultures; Fig. 2*C*). In contrast a thin type I collagen band was observed.

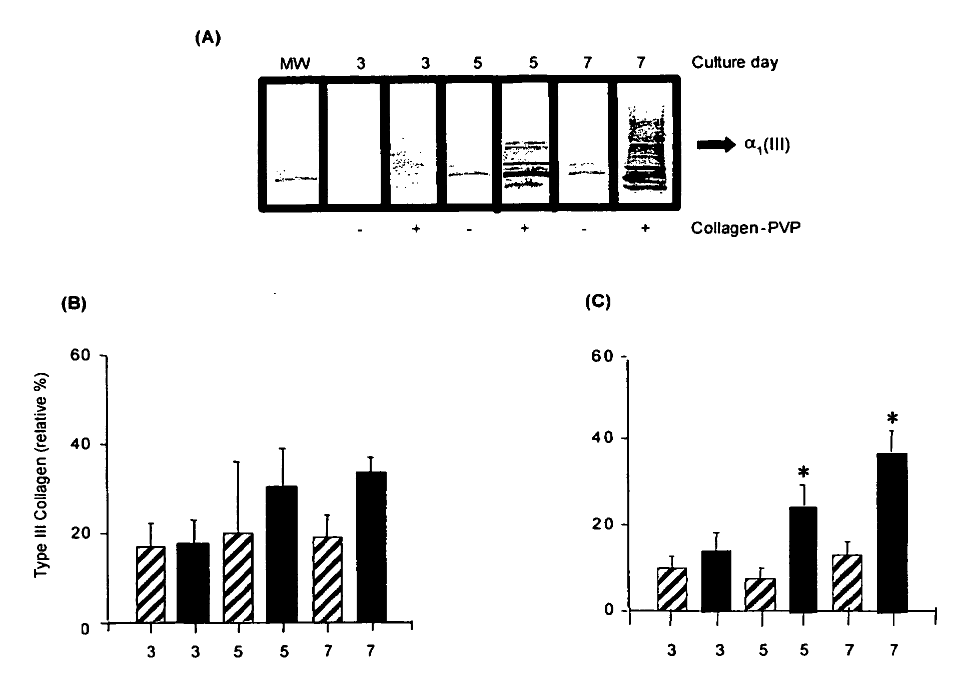

**Fig. 2****.** Collagen-PVP effect on types I and III collagen content in synovial tissue cultures. (A) SDS-PAGE of types I and III collagen of representative RA synovial tissue cultures with (+) or without (-) collagen-PVP at the 3^{rd}, 5^{th} and 7^{th} culture day, respectively. MW: Type I collagen standard. (B) Type III collagen relative percentage in Non-RA synovial tissue cultures. (C) Type III collagen relative percentage in RA synovial tissue cultures. Statistical differences between control and collagen-PVP-treated groups were obtained at the 5^{th} (*p=0.009) and the 7^{th} culture day (*p=0.00007). Data are the mean ± SEM of synovial tissue cultures from 5 Non-RA and 10 RA patients, each done in duplicate.

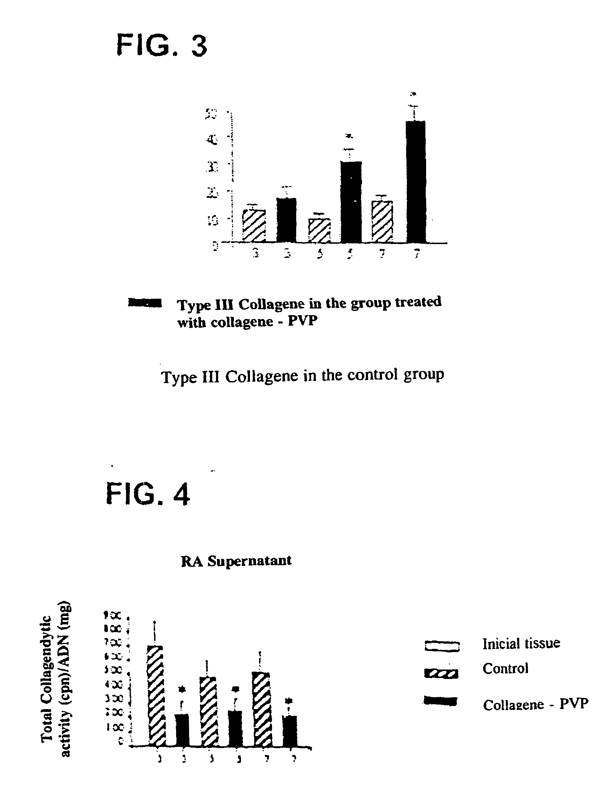

*Collagenolytic activity of supernatants from synovial tissue from RA patients.* During the progression of RA the proteolytic activity at the site of inflammation is increased. Thus, synovia from 10 patients with RA and 5 non-RA patients were examined immunohistochemically in detail using specific antisera to MMP-1. The RA synovia showed a considerable high range in MMP-1 distribution compared with non-RA synovia. There was no difference between treated *versus* control group (Fig. 3*D,E*). On the other hand, in RA supernatants from collagen-PVP-treated cultures, levels of total collagenolytic activity were 1.6-fold lower than those in control cultures (p<0.05; Fig. 3*A*). Calcium-dependent collagenases (MMPs) in supernatants from synovial tissue treated cultures were measured by the difference between the degradation of ³H-collagen in a CaCl₂ buffer and in an EDTA buffer (total collagenolytic activity - calcium-independent collagenases). This proteolytic activity exhibited slightly lower levels in supernatants from biocompound-treated cultures than untreated-ones (Fig. 3*B*). Collagenase activity of proteinases that did not require calcium for their stability (putatively elastase and/or G cathepsin) was 2.2-fold lower compared to untreated tissue cultures (p<0.008; Fig. 3*C*). It is important to highlight that calcium-dependent as well as calcium-independent collagenolytic proteases have similar activity levels. This suggests that in RA pathogenesis, calcium-independent enzymes participate with the same magnitude to that observed for calcium-dependent enzymes.

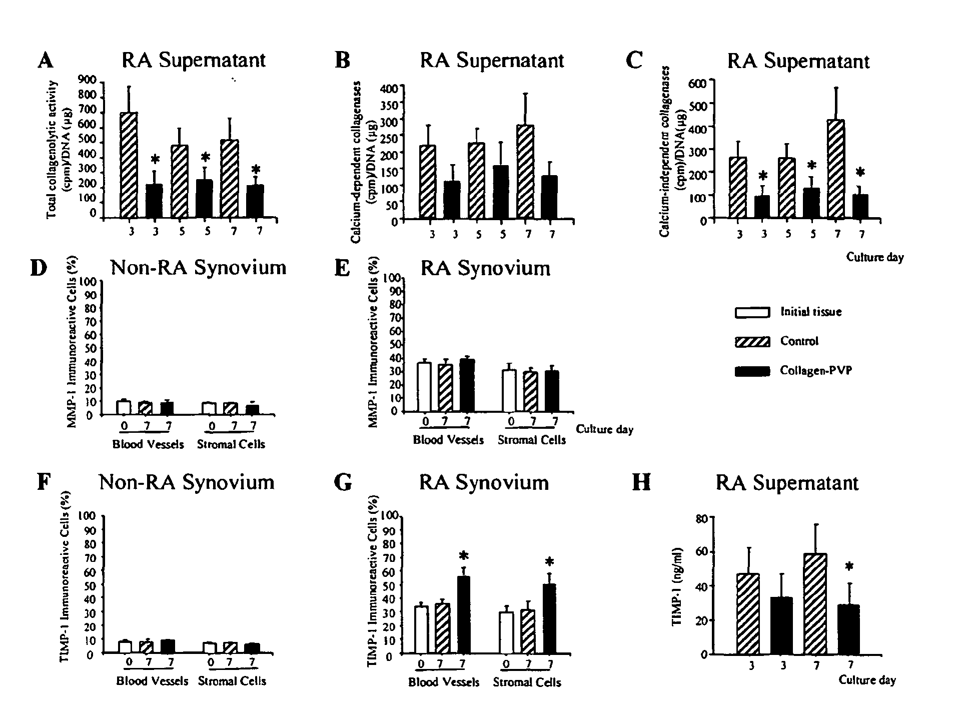

**Fig. 3****.** Collagen-PVP effect on collagenase activity and TIMP-1 expression in synovial tissue cultures. (A) Total collagenolytic activity. Data were statistically significant with *p=0.008, *p=0.055 and *p=0.05 for the 3^{rd}, 5^{th} and 7^{th} culture day, respectively. (B) Calcium-dependent collagenolytic activity. (C) Calcium-independent collagenolytic activity. Collagen-PVP-treated groups were compared with untreated cultures and the differences were statistically significant with *p=0.008, *p=0.006 and *p=0.002 for the 3^{rd}, 5^{th} and 7^{th} culture day, respectively. Collagenase activity was expressed as cpm/24 h/µg of DNA at 35°C. Data are mean ± SEM of synovial tissue cultures from 10 RA patients, each done in duplicate. (D) MMP-1 immunoreactive cells on non-RA synovial tissue. (E) MMP-1 immunoreactive cells on RA synovial tissue. (F) TIMP-1 immunoreactive cells on non-RA synovial tissue. (G) TIMP-1 immunoreactive cells on RA synovial tissue. Data are mean ± SEM of synovial tissue cultures from 5 non-RA and 10 RA patients, each done in duplicate where of each tissue at least two sections were evaluated. (H) TIMP-1 concentration in synovial tissue supernatant with or without collagen-PVP treatment (*p=0.04 at 7^{th} culture day). Data are the mean ± SEM of synovial tissue cultures from 10 RA patients, each done in duplicate.

*TIMP-1 concentration in synovial tissue cultures from RA patients.* Sections of RA synovium stained with anti-TIMP-1 showed strong immunoreactivity in blood vessel and stromal cells in collagen-PVP-treated synovial tissue from RA patients (Fig. 3*G*). Collagen-PVP has not any effect on synovium from non-RA patients (Fig. 3*F*). However, TIMP-1 levels in supernatants from control cultures contained 1.7-fold higher levels of the glycoprotein than treated cultures at the 7^{th} day (p=0.04; Fig. 3*H*).

*Adhesion molecule expression in synovial tissue cultures from non-RA and RA patients.* In order to establish whether ICAM-1 and VCAM-1 molecules, inflammatory markers, were modified by collagen-PVP treatment, they were detected in synovium. The ICAM-1 expression in cultures from non-RA patients was similar between control and collagen-PVP-treated group (11-17%; Table 2). However, in treated cultures from RA patients, both ICAM-1 and VCAM-1 molecules showed lower levels of intensity and immunoreactivity than control cultures (Table 2). The levels were statistically significant for ICAM-1 at the 7^{th} culture day in blood vessels (p=0.03) and stromal cells (p=0.04) and the percentage of positive ICAM-1 cells from treated cultures was similar to that determined for normal synovial tissue cultures (Table 2) [30]. In addition, VCAM-1 expression in blood vessels and stromal cells from treated cultures also showed a substantial down-regulation (p<0.05; Table 2).

**Table 2. Collagen-PVP effect on molecule expression on blood vessel cells and stromal cells from synovial tissue cultures from non-RA and RA patients.**

| | | Non-RA | | | RA | | |
|---|---|---|---|---|---|---|---|
| | Culture day | 0 | 7^{th} | 7^{th} | 0 | 7^{th} | 7^{th} |
| | Treatment | - | - | + | - | - | + |
| Antibody to: | Cell type: | | | | | | |
| ICAM-1 | Blood vessel | 12.0±0.8 | 11.6±0.9 | 13.0±0.7 | 64.0±4.8 | 57.3±10.6 | 29.2±15.2* |
| | Stroma | 11.0±1.8 | 10.7±1.1 | 10.0±1.2 | 28.6±14.0 | 31.0±11.4 | 18.3±11.0* |
| VCAM-1 | Blood vessel | 16.0±3.5 | 17.0±1.6 | 14.8±0.9 | 53.5±6.7 | 48.6±7.0 | 21.4±13.1* |
| | Stroma | 15.6±0.2 | 16.0±0.8 | 15.3±1.4 | 36.1±11.2 | 38.9±11.6 | 17.1±7.7* |
| | Blood vessel | 7.9±0.6 | 8.7±1.0 | 8.0±1.8 | 26.4±20.2 | 27.4±19.0 | 10.4±6.3 |
| IL-6 | Stroma | 7.3±1.5 | 8.5±2.0 | 9.0±2.7 | 12.2±10.6 | 14.5±9.0 | 6.1±3.8 |
| | Inflammatory | 8.0±1.5 | 8.3±2.6 | 7.6±2.0 | 57.7±17.1 | 58.5±10.0 | 20.0±2.5* |

| Cox-1 | | | | | | | |
|---|---|---|---|---|---|---|---|
| FAS/ | Blood vessel | 22.0±1.5 | 23.4±6.1 | 21.8±3.8 | 20.2±5.4 | 20.2±9.9 | 55.2±12.5* |
| Apo95 | Stroma | 15.5±4.6 | 19.8±5.2 | 20.0±4.2 | 22.0±5.2 | 26.1±8.9 | 17.5±3.7 |
| TUNEL | Blood vessel | 14.9±5.2 | 14.4±3.8 | 16.0±5.3 | 18.1±7.74 | 13.9±2.9 | 54.9±4.7* |
| | Stroma | 13.5±2.8 | 14.5±3.7 | 14.5±3.6 | 13.8±4.8 | 14.7±4.3 | 35.6±9.7* |

| | | | | | | | |
|---|---|---|---|---|---|---|---|
| ^{†}The results depict mean ± SD of molecule expression (percentage of immunoreactive cells determined by immunohistochemistry) in synovial tissue cultures from 5 non-RA and 10 RA patients each done in duplicate where of each tissue at least two sections were evaluated. (-) without collagen-PVP; (+) with 1% collagen-PVP; (ND) Not Done; (*) p<0.05; 0 *vs.* 7^{th} culture day. | | | | | | | |

*Proinflammatory cytokine production by synovial tissue cultures.* In RA many factors are involved in synovial inflammation, where the cytokines such as IL-1, TNF-α, IL-6 and IL-8, have emerged as regulatory factors of particular importance. In order to establish the effect of collagen-PVP on the expression of these cytokines, we determined these proteins in the synovium by immunohistochemistry. Results showed that IL-8 was expressed at significantly higher levels in non-treated synovial tissue cultures from RA patients than in non-RA and RA treated cultures (Fig. 4*A*, *B*). Meanwhile IL-6 did not show any difference between RA treated cultures and control cultures (Table 2).

Since collagenolytic activity, CAMs and TIMP-1 expression levels were down-modulated with collagen-PVP treatment, we suspected that the production of IL-1β and TNF-α was modified.

The exogenous addition of the biodrug to non-RA tissue cultures did not produce any effect on TNF-α expression in blood vessels nor stromal cells (Fig. 4*C*). TNF-α expression was down-modulated by collagen-PVP in tissue from RA patients at statistical significant levels compared with non-treated cultures (p<0.05; Fig. 4*D*). Moreover, collagen-PVP down-regulated TNF-α protein concentration (4-fold) in supernatants (p<0.02; Fig. 4*E*). The same down-regulated pattern of IL-1β expression was observed in RA tissue treated with collagen-PVP (p<0.05; Fig. 4*F*) and in supernatants (3-fold; p<0.03; Fig. 4*G*).

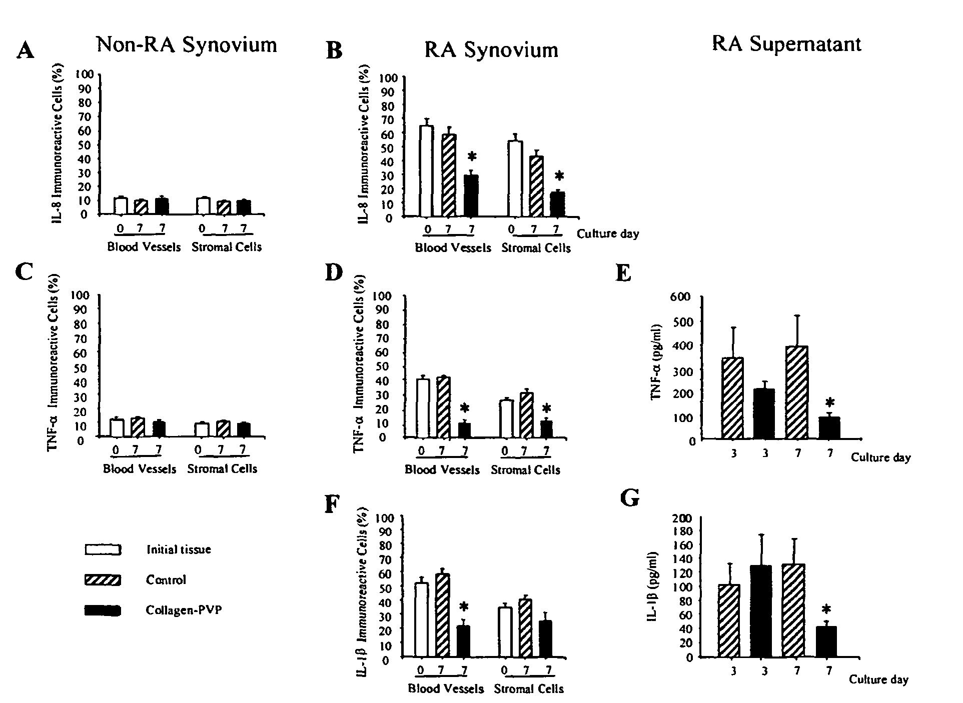

**Fig. 4**. Effect of collagen-PVP on proinflammatory cytokine expression in synovial tissue cultures. (A) IL-8 immunoreactive cells on blood vessels and stromal cells from non-RA patients. (B) IL-8 immunoreactive cells in synovial tissue from RA patients (*p<0.05). (C) TNF-□ immunoreactive cells on blood vessels and stromal cells from non-RA patients. (D) TNF-□ immunoreactive cells on blood vessels and stromal cells from RA patients (*p<0.05). Data are mean ± SEM of synovial tissue cultures from 5 non-RA and 10 RA patients, each done in duplicate where of each tissue at least two sections were evaluated. (E) TNF-□production (*p=0.03) at 7^{th} day. Data are the mean ± SEM of synovial tissue cultures from 10 RA patients, each done in duplicate. (F) IL-1□ immunoreactive cells on blood vessels and stromal cells from RA patients (*p<0.05). Data are mean ± SEM of synovial tissue cultures from 5 non-RA and 10 RA patients, each done in duplicate where of each tissue at least two sections were evaluated. (G) IL-1□ concentration in supernatants from RA synovium cultures (*p=0.05). Data are the mean + SEM of synovial tissue cultures from 10 RA patients, each done in duplicate.

*Effect of collagen-PVP on Cox-1 expression.* Cox-1 is a constitutively enzyme that synthesized prostaglandins pathway arachidonic acid metabolism. Prostaglandins likely contribute to synovial inflammation by increasing local blood flow and potentiating the effects of mediators such as bradykinin and IL-1 that induce vasopermeability. In this vein, the immunohistochemistry showed that collagen-PVP induced a negative modulation on the expression of Cox-1 in RA tissue compared with untreated cultures (Table 2). However, biodrug did not show effect on non-RA tissue (Table 2).

*Expression of FaslApo95 and detection of DNA strand breaks in apoptotic synovial cells by in situ nick translation.* It has been shown that Fas antigen is expressed on the surface of synovial cells and mediates cell death of the Fas expressing synovial cells when stimulated with agonistic anti-Fas. However, defective apoptosis is intimately associated with RA thus, the function of the Fas/FasL system seems to be incapable of eliminating cells in the proliferative RA synovium. Due to, we examined whether Fas antigen was expressed on synovium as well as the presence of apoptotic cells. We found that Fas/Apo95 was predominantly up-regulated on blood vessel cells from RA synovium collagen-PVP-treated (Table 2). When we applied the *in situ* cell death detection assay for RA synovial tissues to detect apoptotic cells, TUNEL technique showed an up-regulation mostly in blood vessel and stromal cells (50%) (Table 2) treated with collagen-PVP *(*Furuzawa-Carballeda J, Rodríguez-Calderón R, Diaz de León L, Alcocer-Varela J Mediators of inflammation are down-regulated while apoptosis is up-regulated in rheumatoid arthritis synovial tissue by polymerized collagen. Clin Exp Immunol 2002;130:140-9*)*.

### Phase I Clinical Trial in Rheumatoid Arthritis (RA) Patients (not part of the invention)

*Patients.* Demographic and clinical characteristics of patients at the time on their basal visit and last dose of collagen-PVP are summarized in Table 3.

**Table 3. Demographic and disease history at baseline and 3 months of study.**

| Variable | | Baseline | 3-months |
|---|---|---|---|
| Demographics | Sex (FIM) | 11/0 | 1010* |
| | Age (mean±SD), years | 45.7 ± 12.8 | 45.7 ± 12.8 |
| | Range | 27-68 | 27-68 |
| | Disease duration (mean±SD), years | 12.1 ± 9.6 | 12.1 ± 9.6 |
| | Range | 6-33 | 6-33 |
| Clinical variables | DAS (mean±SD) | 4.6 ± 1.1 | 3.2 ± 0.83** |
| | Range | 2.55-6.16 | 1.9-4.6 |
| | No. of swollen joints (mean±SD) | 14.9 ± 8.1 | 4.2 ± 4.2** |
| | Range | 7-35 | 1-15 |
| | Ritchie index (mean±SD) | 22.0 ± 12.2 | 11.8 ± 6.5** |
| | Range | 11-45 | 5-26 |
| | Duration of morning stiffness (mean±SD), min | 47.1 ± 28.2 | 14.8 ± 29.3** |
| | Range | 5-75 | 0-90 |
| | Visual analog scale (mean±SD), mm | 62.5 ± 12.9 | 22.6 ± 19.4** |
| | Range | 40-75 | 0-56 |
| | HAQ (mean+SD) | 1.1 ± 0.3 | 0.6 ± 0.6** |
| | Range | 0.65-1.6 | 0.05-1.7 |
| Laboratory | Hemoglobin (mean±SD), g | 13.3 ± 1.6 | 13.6 ± 1.6 |
| | Range | 10.6-15.6 | 11.2-16.3 |
| | Platelets (mean±SD), K/µl | 293.2 ± 106.7 | 325.9 ± 108.2 |
| | Range | 98.0-447.0 | 121.0-458.0 |
| | ESR (mean±SD), mm/h | 31.1 ± 23.1 | 30.2 ± 26.0 |
| | Range | 1-59 | 2-81 |
| | RF positive (%) | 90.0 | 90.0 |
| | CRP (mean±SD), mg/l | 2.3 ± 3.5 | 1.93 ± 2.3 |
| | Range | 0.1-11.9 | 0.1-7.8 |
| NSAID used after baseline | Paracetamol (mean±SD), tablets/week | 7.8 ± 7.1 | 8.6 ± 10.1 |
| | Range | 0.0-21.0 | 0.0-32.0 |

| | | | |
|---|---|---|---|
| *Premature discontinuation on 7^{th} week. HMR patient had a final clinical and laboratory examination and a set of questionnaires to answer. **p<0.05 | | | |

*Safety.* Collagen-PVP was safety and well tolerated during extended therapy and adverse events were not detected, except pain during 5 min in the injection site.

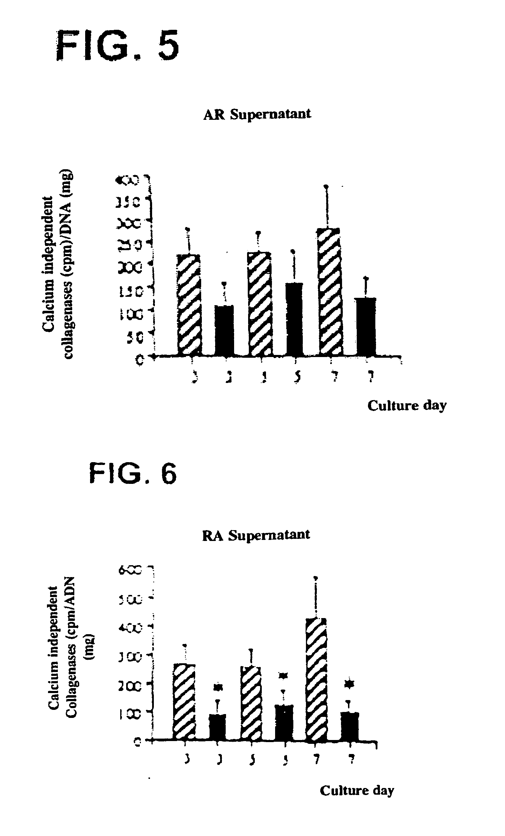

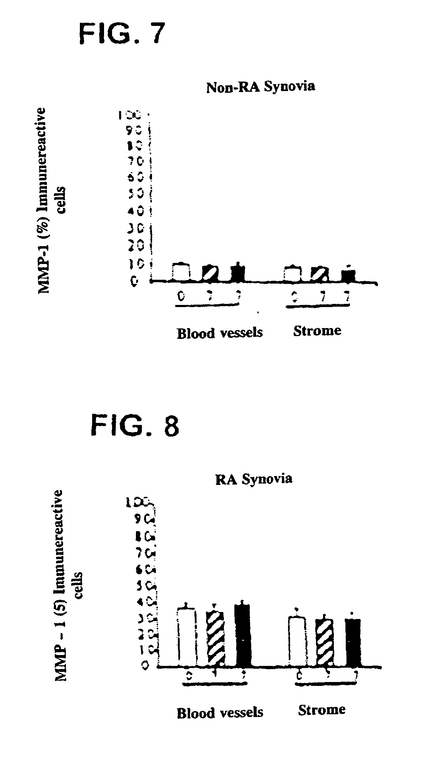

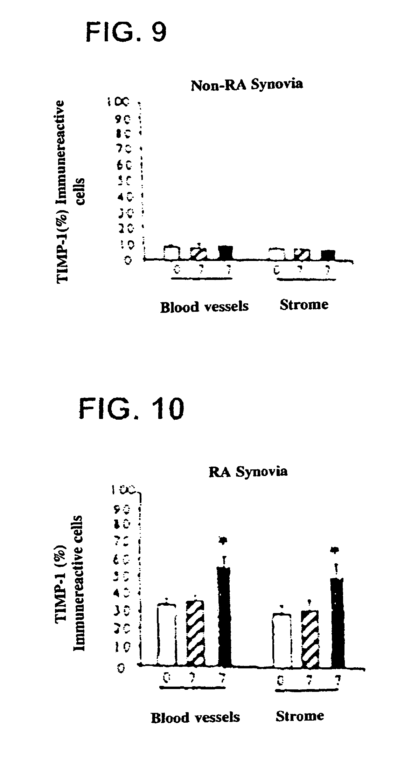

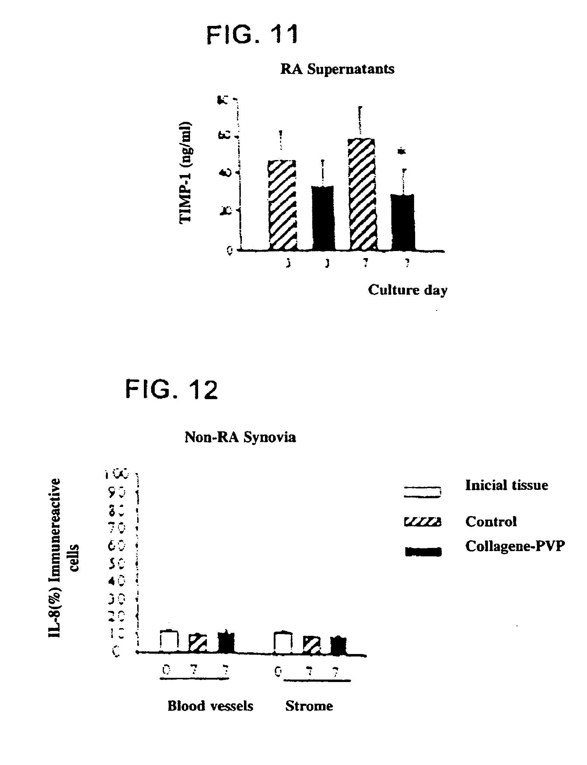

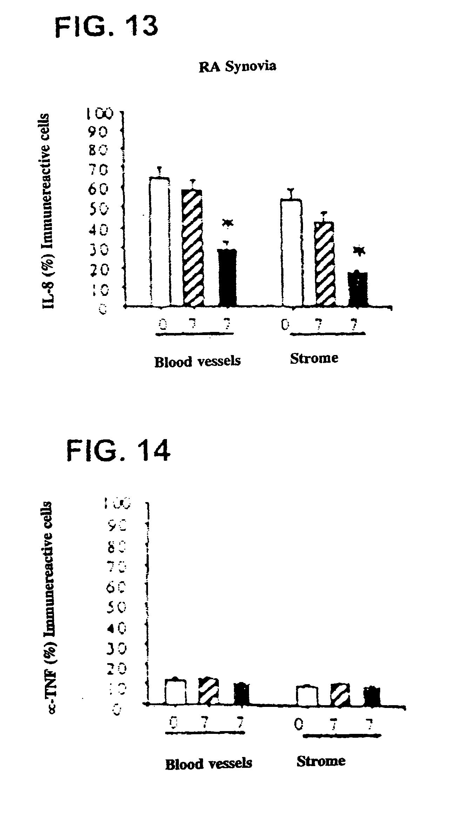

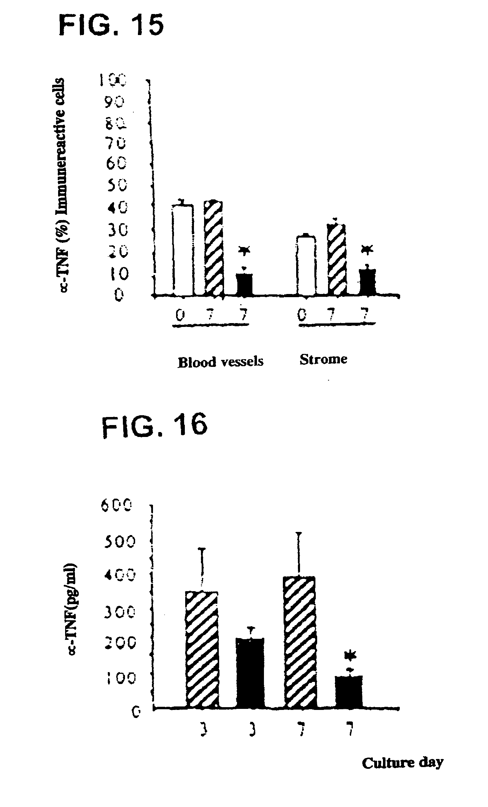

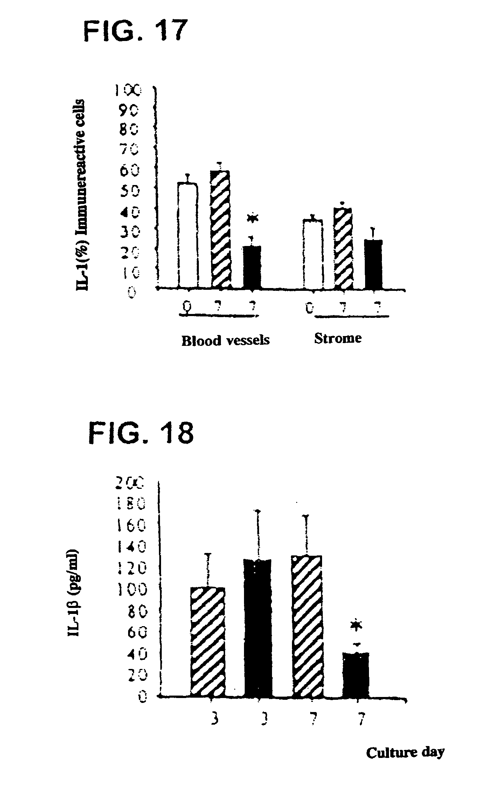

*Efficacy and clinical benefit.* Subcutaneous administration of collagen-PVP during 3 months in the 8 painful joints showed that swollen and tender joint (Ritchie Index or RI) counts (Fig. 5) have improved rapidly (RI: Δ-10.2 ≅ -46.4%; swollen joint count: Δ-10.7 ≅ -71.8%). This improved was sustained during long-term therapy.

Besides, very similar and highly significant differences in changes in other variables of disease activity such as morning stiffness and visual analogue scale pain (morning stiffness: Δ-32.3 ≅ - 68.6%; VAS: Δ-39.9 ≅ -63.8%; Fig 5).

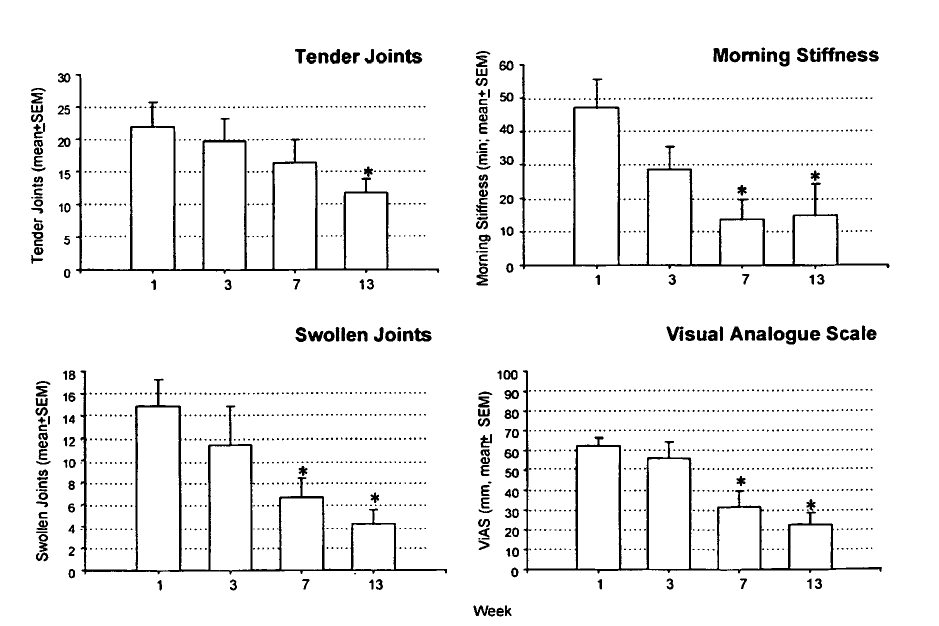

***Fig.* 5**. Changes in clinical manifestations during the treatment course with collagen-PVP. A collagen-PVP dosage of 1.7 ml produced a sustained reduction of active joints. Tender joint count: *p=0.02, 1 *vs.* 13 weeks; Swollen joint count: *p=0.007, I *vs.* 7 weeks; *p=0.001, 1 *vs.* 13 weeks; Morning stiffness: *p<0.009, 1 *vs.* 7 weeks and *p<0.02, 1 *vs.* 13; Pain: *p=0.007, 1 *vs.* 7 weeks; *p=0.001, 1 *vs.* 13 weeks. 1=first visit or baseline; 3=Third visit and 2^{nd} subcutaneous application of collagen-PVP; 7=Seventh visit and 6^{th} subcutaneous injection of collagen-PVP; 13=Visit No 13 and 12^{th} subcutaneous application.

Patients also had a statistically significant improvement in disease activity score (DAS) (DAS: Δ-1.35 ≅ -70.5%, p<0.02). The improvement was also determined using twenty, fifty and seventy percent American College of Rheumatology response criteria (ACR20, ACR50 and ACR70). The ACR20 was achieved by 80.0% of patients at 3 months (8 from 10 patients). Similarly, 60.0% of patients achieved the ACR50 (6 from 10 patients) and 20.0%, the ACR 70 (2 from 10 patients) at this time (Fig. 6). The physical functional status (Spanish-HAD-DI), also showed a significant difference in change from their baseline values and 3 months of collagen-PVP treatment (HAQ-DI: Δ-0.5 ≅ -48.5%; p<0.05, 1 *vs.* 13 weeks; Fig.6).

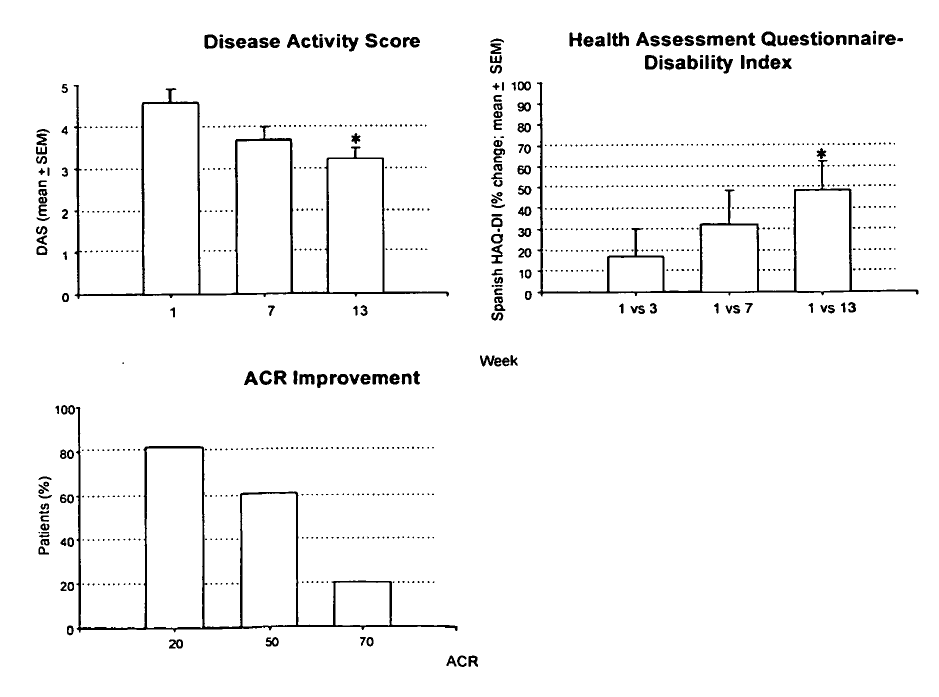

***Fig, 6******.*** Response to therapy evaluated by Disease Activity Score (DAS), Health Assessment Questionnaire-Disability Index (HAQ-DI) and American College of Rheumatology (ACR) criteria. DAS: *p=0.015, 1 *vs.* 13 weeks and HAQ-DI: *p=0.045, 1 *vs*. 13 weeks. 1=first visit or baseline; 3=Third visit and 2^{nd} subcutaneous application of collagen-PVP; 7=Seventh visit and 6^{th} subcutaneous injection of collagen-PVP; 13=Visit and 12^{th} subcutaneous application.

*Concomitant medication.* All patients have not modified their oral methotrexate and dosage of concomitant NSAIDs during 3 months of the study (Table 3).
*Laboratory assessment.* There was no change in Erythrocyte Sedimentation Rate, hematological constants (hemoglobin, hematocrit, blood chemistry, red blood cells count, leukocytes and platelets), neither C Reactive Protein nor Rheumatoid Factor. None of the patients were positive for antibodies to double chain of DNA and Ribonucleoprotein at baseline and after treatment.
Radiological analysis was no different at baseline and after treatment. However, is important to mention that there was no narrowing joint space, bone erosion nor cartilage degradation *(*Furuzawa-Carballeda J, Cabral AR, Zapata-Zúñiga M, Alcocer-Varela J. Subcutaneous administration of polymerized-type I collagen for the treatment of patients with rheumatoid arthritis. J Rheumatol 2003;30:256-9*).*

*In vitro* study evaluated the anti-inflammatory and apoptotic effect of polymerized type I collagen (collagen-PVP), as well as collagen turnover in synovial tissue from RA patients.
Based on these results, we suggest that collagen-PVP added to RA synovium cultures, modulates collagen turnover, since the biodrug decreases collagenolytic activity, as well as TIMP-1 production, and increases the amount of type III collagen and TIMP-1 to similar levels observed in normal synovium. The chronic inflammatory process is altered by collagen-PVP action, as described previously by Krötzsch-Gómez *et al.,* and Barile *et al., (*Krötzsch-Gómez FE, Furuzawa-Carballeda J, Reyes-Márquez R, Quiróz-Hernández E, Diaz de León L. Cytokine expression is downregulated by collagen-polyvinylpyrrolidone in hypertrophic scars. J Invest Dermatol 1998;111:828-34*;* Barile L, Furuzawa-Carballeda J, Krötzsch-Gómez FE, Espinosa-Morales R, Alcalá M, Diaz de León L. Comparative study of collagen-polyvinylpyrrolidone vs.. triamcinolone acetate in systemic sclerosis. Clin Exp Rheumatol 1998;16:370*),* presumably due to the down-regulation of IL-1β, TNF-α, IL-8 as well as cell adhesion molecules and inducing Cox- 1 activation. Besides cell binding through integrin-extracellular matrix interaction generates signals that regulate MMP expression *(*Larjava H, Lyons J, Salo R, Makela M, Koivisto L, Birkedal-Hansen H, Akiyama SK, Yamada KM, Heino J. Anti-integrin antibodies induce type IV collagenase gene expression in keratinocytes. J Cell Physiol 1993;157:190-200*;* Romanic AM, Madri JA. The induction of 72 kD gelatinase in T cells upon adhesion to endothelial cells is VCAM-1 dependent. J Cell Biol 1994;125:1165-78*;* Tremble P, Chiquet-Ehrismann R, Werb Z. The extracellular matrix ligands fibronectin and tenascin collaborate in regulating collagenase gene expression in fibroblasts. Mol Biol Cell 1994;5:4390-3*;* Rükonen T, Westermarck J, Koivisto L, Broberg A, Kähäri VM, Heino. Integrin □2□1 is a positive regulator of collagenase (MMP-1) and collagen □1(I) gene expression. J Biol Chem 1995; 270:13548-52*)* suggesting that cell adhesion molecules can also participate in RA tissue destruction. Also, down production of IL-1β and TNF-α seems to stimulate synovial cell death via apoptosis in synovium cultures, the last may contributed to inhibit the outgrowth of synovial cells that eventually leads to hyperplasia or pannus formation and the destruction of RA joints *(*Furuzawa-Carballeda J, Rodriguez-Calderón R, Diaz de León L, Alcocer-Varela J. Mediators of inflammation are down-regulated while apoptosis is up-regulated in rheumatoid arthritis synovial tissue by polymerized collagen. Clin Exp Immunol 2002;130:140-9*).*
In conclusion, we showed that the addition of collagen-PVP to synovial tissue cultures induced a down-modulation but not an inhibition of inflammatory parameters in rheumatoid synovium.
This study was seminal to consider the collagen-PVP as a coadjuvant for the RA treatment. The last affirmation was based on the knowledge that type I collagen is phylogenetically, one of the oldest extracellular matrix proteins. It possesses a very low antigenicity and it is relatively easy to metabolize. Furthermore, it is able to stimulate cells to form new tissues, it means, collagen has informational character *(*Pohunlová H, Adam M Reactivity and the fate of some composite bioimplants based on collagen in connective tissue. Biomaterials 1995;16: 67-71*).* In 1981, the Food and Drug Administration (FDA) approved the use of an injectable form of reconstituted purified bovine collagen (Zyderm; Collagen Corp., Palo Alto, CA) as a medical device for augmenting the volume of soft tissues *(*Clark DP, Hanke CW, Swanson NA. Dermal implants: Safety of products injected for soft tissue agumentation. J Am Acad Dermatol 1989;21:992-8*).*

Current interest in this injectable form of collagen is focused on defining broader clinical applications and the development of new products.
Until now, most of the injectable collagen or collagen implants were derived from bovine dermal or tendinal atelopeptide collagen (Zyderm, Zyplast and Atelocollagen) *(*Soo Ch, Rahbar G, Moy RL. The immunogenicity of bovine collagen implants. J Dermatol Surg Oncol 1993;19:431-434*;* Charriere G, Bejot M, Schnitzler L, Ville G, Hartmann DJ. Reactions to a bovine collagen implant. J Am Acad Dermatol 1989; 21:1203-1208*).* However, the present study used polymerized- porcine type I collagen. It has anti-inflammatory properties *(*Krötzsch-Gómez FE, Furuzawa-Carballeda J, Reyes-Márquez R, Quiróz-Hernández E, Diaz de León L. Cytokine expression is downregulated by collagen-polyvinylpyrrolidone in hypertrophic scars. J Invest Dermatol 1998;111:828-34*;* Barile L, Furuzawa-Carballeda J, Krötzsch-Gómez FE, Espinosa-Morales R, Alcalá M, Diaz de León L. Comparative study of collagen-polyvinylpyrrolidone vs.. triamcinolone acetate in systemic sclerosis. Clin Exp Rheumatol 1998;16:370*).* Collagen-PVP which is made pepsinized porcine type I dermal collagen (atelopeptidic) due to it is a week immunogen. We preferred, porcine type I collagen due to pigs are among the primary animal species proposed as sources for xenografts in a variety of practical, ethical, and safety reasons including the high homology with human collagen *(*Weiss RA. Xenografts and retroviruses. Science 1999; 285:1221-1222*;* Paradis K, Langford G, Long Z, Heneine W, Sandstrom P, Switzer WM, Chapman LE, Lockey Ch, Onions D, Otto E. Search for cross-species transmission of porcine endogenous retrovirus in patients treated with living pig tissue. Science 1999;1236-1241*;* Beard HK, Faulk WP, Conochie LB, Glynn LE. Some immunological aspects of collagen. Progr Allergy 1977;22:45-106*).*
On the other hand, electrophoretic data suggest that collagen-PVP binds covalently to PVP. The covalent binding with PVP conveys both increased collagen stability and reduced collagen antigenicity. Because of its unique chemical nature, low molecular weight makes PVP biologically inert and safe *(*Clark DP, Hanke CW, Swanson NA. Dermal implants: Safety of products injected for soft tissue agumentation. J Am Acad Dermatol 1989;21:992-8*;* Soo Ch, Rahbar G, Moy RL. The immunogenicity of bovine collagen implants. J Dermatol Surg Oncol 1993;19:431-434*).* Besides, the homopolymer of N-vinyl-2-pyrrolidone or PVP confers on the biodrug pharmaceutical properties different than those observed in collagen or PVP alone.

In contrast to bovine collagen implants, we did not find any adverse side effect with collagen-PVP, such as those previously described (hypersensitivity reactions, mild erythema, urticaria, localized swelling, and specific anti-type I collagen antibody production) *(*McClelland M, DeLustro F. Evaluation of antibody class in response to bovine collagen treatment in patients with urinary incontinence. J Urol 1996;155:2068-73*;* DeLustro F, Fries J, Kang A. Immunity to injectable collagen and autoimmune disease: a summary of current understanding. J Dermatol Surg Oncol 1988;14(Suppl I):57-65*;* Singh G, Fries JF. Autoimmune disease and collagen dermal implants. Ann Int Med 1994;120:524*;* Lewy RI. Autoimmune disease and collagen dermal implants. Ann Int Med 1994;120:525*).* At difference of the study of Sieper, et al., (1996) with collagen administration we did not find any adverse effect after administration of collagen-PVP nor elevation in laboratory test (C reactive protein, erythrocyte sedimentation rate nor rheumatoid factor) *(*Sieper J, Kary S, Sörensen H, Alten R, Eggens U, Hüge W, Hiepe F, Kühne A, Listing J, Ulbrich N, Braun J, Zink A, Mitchison NA. Ordl type II collagen treatment in early rheumatoid arthritis. A double-blind, placebo-controlled, randomized trial. Arthritis Rheum 1996;39:41-9*).*
The collagen-PVP administration scheme was consistent with Tarkowski's scheme (1999) who demonstrated that the effect of 3 daily *vs.* 3 weekly treatments with equivalent doses of cholera-toxin B subunit-type II collagen conjugate on days 7, 14, 21 after disease induction in mice diminished significantly then incidence of arthritis compared with mice treated daily with unconjugated type II collagen or a control cholera toxin B *(*Tarkowski A, Sun J-B, Holmdahl R, Holmgren J, Czerkinsky C. Treatment of experimental autoimmune arthritis by nasal administration of a type II collagen-cholera toxoid conjugate vaccine. Arthritis Rheum 1999;42:1628-34*).*
In accordance with doses (1.7 mg of collagen), administration pathway (subcutaneous), and the frequency of administration, we suggest that collagen-PVP could act also, like a tolerance-inducing-molecule more effective than the other ways of administration and with less toxic effects. Based on the background of collagen-PVP, we think that it generates cytokine-secreting regulatory cells more than anergy *(*Furuzawa-Carballeda J, Cabral AR, Zapata-Zúñiga M, Alcocer-Varela J. Subcutaneous administration of polymerized-type I collagen for the treatment of patients with rheumatoid arthritis. J Rheumatol 2003;30:256-9*).*
Using ACR criteria, the 20% response rate with 1.6 ml of collagen-PVP was 80.0% at month 3. Good results were seen with more demanding criteria (60.0% response at ACR50 and a 20.0% response at ACR70). These values were similar to that obtained by etanercept in a phase II trial *(*Moreland LW, Baumgartner S, Schiff MH, et al. Treatment of rheumatoid arthritis with a recombinant human tumor necrosis factor receptor (p75)-Fc fusion protein. N Engl J Med 1997;337:141-7*).*
In most cases, there was a continuous improvement over the 12^{th} week, this could be taken as evidence that a longer treatment period might be more favorable.
Given these encouraging results and the fact that this therapy has no known side effects further studies evaluating its effectiveness, optimal doses, mechanism of action, a placebo controlled study and other pathways of administration are warranted.

**Collagen-PVP for the treatment of patients with rheumatoid arthritis. Effect of intramuscular administration in a double blind placebo-controlled study (not part of the invention)** The study was a prospective, longitudinal, double blind placebo-controlled and included 30 patients with active RA (ACR). Patients on stable doses of methotrexate were treated in a Freyberg scheme with intramuscular injections of 0.4 ml of collagen-PVP (3.33 mg of collagen) or 0.4 ml of placebo during 6 months. The primary endpoints included disease activity score (DAS) and improvement was determined using American College of Rheumatology response criteria (ACR20, 50 and 70) that refers a mean change in 3 from 5 evaluations including the Ritchie index (RI), swollen joint count, Spanish-health assessment questionnaire (HAQ-DI), pain intensity on a visual analogue scale (VAS) and erythrocyte sedimentation rate (ESR) or C-reactive protein (CRP). Statistical analysis was performed using double tail Mann Whitney U-test. Collagen-PVP was safe and well tolerated. Patients had a statistically significant improvement (p<0.05) in collagen-PVP-treated *vs*. placebo at 6 months of treatment in: swollen joint count (8.1±3.7*vs*.17.9±4.8), RI (10.7±3.3*vs*.17.8±5.4), morning stiffness (9.4±4.6*vs*.37.8±6.7), Rheumatoid factor (207.7±179.1*vs*.518.8±293.2), C reactive protein (2.7±3.9*vs*.28.1±24.5), HAQ (46.7±15.6*vs*.18.8±13.7), DAS (3.5+0.7*vs*.4.8±0.9), ACR20 (100vs.77.8%), ACR50 (67.6*vs.*11.1%) y ACR70 (33.3*vs.*0%). Serological or hematological parameters remained unchanged. There were no adverse events.

### Conclusions

Collagen-polyvinylpyrrolidone exhibits an important downmodulatory effect on inflammation, on apoptosis induction, as well as collagen turnover in the synovial tissue of the patients suffering AR, perhaps through peripheral immune tolerance.
This copolymer modifies, more cellular metabolism than proliferation. This is suggested since type III collagen relative rate is increased, meanwhile type I collagen diminishes in the synovia treated with the drug, although total content of collagen is not modified. Also, collagen-polyvinylpyrrolidone reduces proteolityc activity, due to calcium independent collagenases, modifying ECM protein turnover, mainly collagen and reestablishing the content of type III collagen.
The information expressed above suggests that collagen-polyvinylpyrrolidone induction of the reduction of collagenolityc activity could contribute to diminish rheumatoid synovitis secuelae, cartilage and bone invasion by inflammatory cells and their erosion and degradation.
Even more, collagen-polyvinylpyrrolidone induces the increase of TIMP-1 in AR tissues, suggesting that ECM turnover could be diminished by a mechanism that involves MMPs blockade by TIMP-1.
In consequence, collagen-polyvinylpyrrolidone avoids inflammatory cell invasion and fibrosis, due to the drug downmodulates cell adhesion molecules and this effect prevents cell-cell attachment.
According to this information, we suggest that collagen-polyvinylpyrrolidone immunomodulatory effects are focused to modulate adhesion molecule expression in stromal and endothelial cells through the leukocyte traffic reduction to the synovial joint.
Finally, in AR the copolymer downmodulates three pro-inflammatory cytokines: IL-8, TNF-α and IL-β. Physiologically they can induce Cox-1 activation, an in consequence they can increase PGE₂ synthesis, as well as joint cartilage and bone resorption.
Also, the previously mentioned cytokines, have been related to the beginning and AR sickness burst, *in vitro* and *in vivo* in a murine model. It is well known, that TNF-α and IL-1β are capable to induce synthesis and release of several other cytokines, such as GM-CSF, IL-6 and IL-8, and all of them increase MMP production and activity, diminution in ELAM-1, VCAM-1 and ICAM-1 capillary expression in synovia from AR patients.

In conclusion, the subcutaneous treatment of the eight more painful joints with collagen-polyvinylpyrrolidone to patients suffering AR, when they are receiving low doses of metotrexate and/or NSAIDs, improves sickness index (DAS) and an important change in at least 3 of the 5 following measures (ACR20), Ritchie index (painful joints), inflamed joint evaluation, general assessment of the patient about the sickness activity (HAQ), the relative pain scale (EVA), as well as acute phase proteins. Collagen-polyvinylpyrrolidone treatment does not stimulate changes in blood or serum and the copolymer exhibits the advantage that is a biological copolymer that, until now, does not produce any risk for the patient, nor stimulate anti-type I collagen or anti-collagen-polyvinylpyrrolidone antibodies or anti SSA/SSB or anti-DNA antibodies, even when patients have been treated during long periods (more than 12 months). Collagen-polyvinylpyrrolidone is a safe and an efficient biomolecule for the short term AR treatment.

## Claims

1. Use of a collagen-polyvinylpyrrolidone biocomplex for preparing a drug for the treatment of osteoarthritis.

## Patentansprüche

1. Verwendung eines Kollagen-Polyvinylpyrrolidon-Biokomplexes zur Herstellung eines Medikamentes zur Behandlung von Osteoarthritis.

## Revendications

1. Utilisation d'un biokomplexe de collagène-polyvinylpyrrolidone pour la préparation d'un remède pour le traitement de l'ostéoarthrite.
